(19) Europäisches Patentamt
European Patent Office
Office européen des brevets

(11) Publication number: **0 082 023**
**A2**

(12) **EUROPEAN PATENT APPLICATION**

(21) Application number: 82306708.7

(22) Date of filing: 15.12.82

(51) Int. Cl.³: C 07 D 495/04, A 61 K 31/505, A 61 K 31/55, A 61 K 31/57 // (C07D495/04, 333/00, 239/00)

(30) Priority: 16.12.81 JP 203064/81
25.06.82 JP 109374/82

(43) Date of publication of application: 22.06.83
Bulletin 83/25

(84) Designated Contracting States: BE CH DE FR GB IT LI NL

(71) Applicant: SANKYO COMPANY LIMITED,
No. 1-6, 3-chome Nihonbashi Honcho Chuo-ku, Tokyo (JP)

(72) Inventor: Fukumi, Hiroshi c/o Chemical Research Lab., Sankyo Co., Ltd. 2-58, 1-chome Hiromachi, Shinagawa-ku Tokyo (JP)
Inventor: Saitoh, Fujio c/o Chemical Research Lab., Sankyo Co., Ltd. 2-58, 1-chome Hiromachi, Shinagawa-ku Tokyo (JP)
Inventor: Horikoshi, Hiroyoshi c/o Biological Research Lab., Sankyo Co., Ltd. 2-58, 1-chome Hiromachi, Shinagawa-ku Tokyo (JP)
Inventor: Kobayashi, Shinsaku c/o Biological Research Lab., Sankyo Co., Ltd. 2-58, 1-chome Hiromachi, Shinagawa-ku Tokyo (JP)

(74) Representative: Gibson, Christian John Robert et al, Marks & Clerk 57/60 Lincoln's Inn Fields, London WC2A 3LS (GB)

(54) **Thienopyrimidine derivatives, their preparation and their medical use.**

(57) Compounds of formula (I):

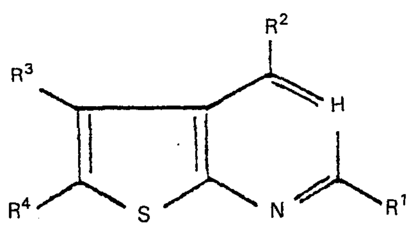

(I)

[wherein $R^1$ represents a variety of aliphatic diamino or triamino groups, a 6- or 7- membered heterocyclic ring containing two nitrogens, one of which is optionally substituted or a tetrahydro-1,4-thiazin-4-yl group; $R^2$ represents an optionally mono- or di- alkyl-substituted amino group, a mono- or di-(2-hydroxyethyl)-substituted amino group or an optionally mono- or di- alkyl-substituted aminoethylamino group; and $R^3$ and $R^4$ each represents hydrogen or $C_1$-$C_4$ alkyl or together represent tri- to penta- methylene]
and salts thereof may be prepared by reacting an equivalent compound, but in which the groups $R^1$ and $R^2$ are replaced by leaving groups, with compounds $R^1H$ (in which free amino groups are optionally protected) and $R^2H$. Various of the compounds have one or more of the following activities: hypoglycaemic activity; the ability to inhibit aggregation of blood platelets; hypotensive activity; or diuretic activity. They can be used in the treatment or prophylaxis of thrombosis, hyperglycaemia, hypertension or oedema and may be formulated as compositions with conventional pharmaceutical carriers or diluents.

EP 0 082 023 A2

THIENOPYRIMIDINE DERIVATIVES, THEIR PREPARATION AND THEIR MEDICAL USE

The present invention relates to a series of new thienopyrimidine derivatives having valuable therapeutic activity, to processes for preparing these derivatives and to their medical use for therapy or prophylaxis.

The thienopyrimidine derivatives to which the present invention relates have been found to have various classes of therapeutic activity, including: hypoglycaemic activity; the ability to inhibit platelet aggregation; diuretic activity and hypotensive activity. Certain of the compounds of the invention exhibit two or more of these classes of activity.

Various thienopyrimidine derivatives are known and certain of these are known to have the ability to inhibit platelet aggregation. Thus, German Offenlegungsschrift No. 2,200,764 (Karl Thomae GmbH) discloses a series of thienopyrimidine derivatives having an amino group at the 2-position and a cyclic amino group, such as a morpholino group, at the 4-position. The compounds disclosed in

this specification are said to have the ability to inhibit the aggregation and adhesion of blood platelets.

A related series of thienopyrimidines is disclosed in U.S. Patent Specification No. 4,146,716 (assigned to Imperial Chemical Industries Limited) and in United Kingdom Patent Specification No. 1,570,494 (Imperial Chemical Industries Limited). These compounds have a variety of substituents at the 2- and 4- positions and are said to have anti-fungal, anti-viral, anti-bacterial and insecticidal activities and are proposed for the treatment of fungal, viral and bacterial infections of plants and for the eradication of insect pests. There is no suggestion that these prior compounds can be used for the treatment of humans or other animals or have any ability to inhibit the aggregation of blood platelets, or have hypoglycaemic, hypotensive or diuretic properties.

We have now discovered a series of new thienopyrimidine derivatives, various of which have hypoglycaemic activity and can be used to treat hyperglycaemia, some of which have the ability to inhibit the aggregation of blood platelets and can thus be used for the therapy or prophylaxis of thrombosis, and some of which have been found to have

hypotensive activity and/or diuretic activity and can be used to treat hypertension and oedema.

The new compounds of the present invention are those compounds which may be represented by the formula (I):

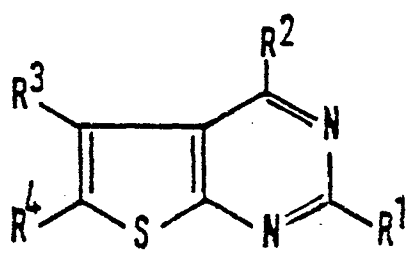

(I)

wherein:

$R^1$ represents an aminoalkylamino group of formula

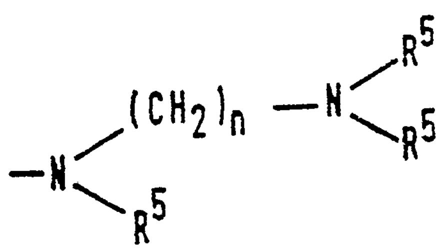

or a bis(aminoalkyl)amino group of formula

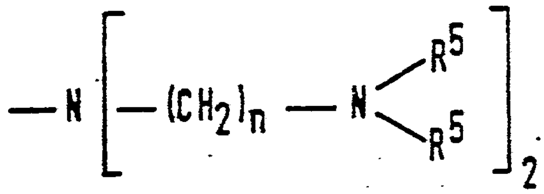

or a heterocyclic group of formula

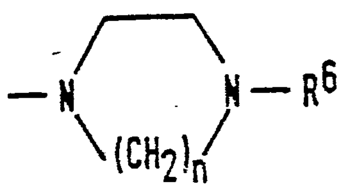

(in which: the symbols $R^5$ may be the same or different and each represents a hydrogen atom or a $C_1$-$C_4$ alkyl group, $R^6$ represents a hydrogen atom, a $C_1$-$C_4$ alkyl group, a formyl group or a $C_2$-$C_4$ alkanoyl group and n is 2 or 3)

or a tetrahydro-1,4-thiazin-4-yl group (also known as the "thiomorpholino group"), which may be represented by the formula

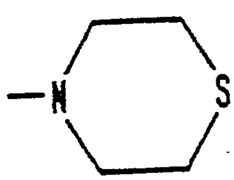 ;

$R^2$ represents an amino group of formula 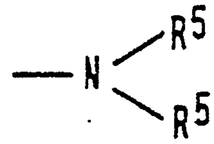

(in which the symbols $R^5$, which may be the same or different, are as defined above), a 2-hydroxyethylamino group, a bis(2-hydroxyethyl)amino group or an aminoethylamino group of formula

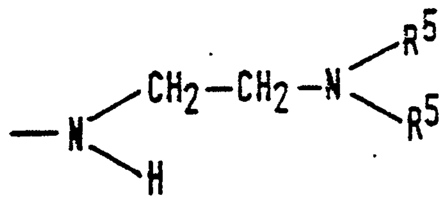

(in which the symbols $R^5$, which may be the same or different, are as defined above); and

$R^3$ and $R^4$ are the same or different and each represents a hydrogen atom or a $C_1$-$C_4$ alkyl group or $R^3$ and $R^4$ together represent a polymethylene group of formula $-(CH_2)_p-$ (in which p is an integer from 3 to 5);

and pharmaceutically acceptable acid addition salts thereof.

The compounds of the invention may be used for the treatment of one or more of the following medical conditions: thrombosis, hyperglycaemia, hypertension and oedema.

The invention also provides a process for preparing a compound of formula (I) or a salt thereof, which process comprises:

(a) reacting a compound of formula (II):

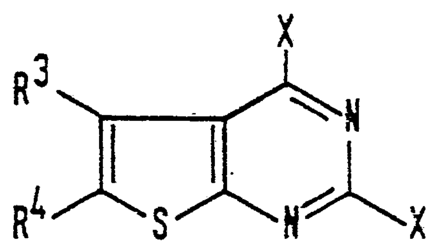

(in which $R^3$ and $R^4$ are as defined above and X represents a halogen atom, a lower alkylsulphinyl group, an arylsulphinyl group, a lower alkylsulphonyl group or an arylsulphonyl group) with a compound of formula $R^2H$ (in which $R^2$ is as defined above), to give a compound of formula (III):

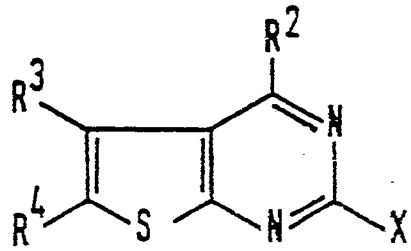

(in which $R^2$, $R^3$, $R^4$ and X are as defined above);

(b) reacting said compound of formula (III) with a compound of formula $R^7H$ (in which $R^7$ represents any one of the groups defined for $R^1$, in which any free

amino group is optionally protected), to give a compound of formula (IV):

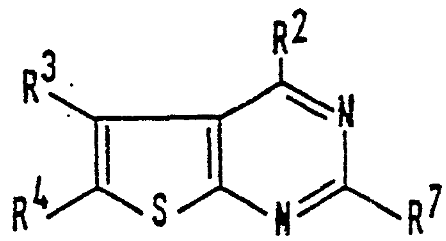

(in which $R^2$, $R^3$, $R^4$ and $R^7$ are as defined above), provided that, where $R^2$ and $R^7$ represent the same groups, the reactions of steps (a) and (b) may be carried out as a single step or as two separate steps;

(c) if necessary, removing any amino-protecting groups to give a compound of formula (I) and, where $R^6$ in said compound of formula (I) represents a hydrogen atom, optionally acylating said compound to give a compound of formula (I) in which $R^6$ represents said formyl or $C_2$-$C_4$ alkanoyl group; and

(d) if necessary, salifying the resulting compound of formula (I).

In the above formula, where $R^3$, $R^4$, $R^5$ or $R^6$ represents a $C_1$-$C_4$ alkyl group, this may be a straight or branched chain alkyl group, for example a methyl, ethyl, propyl, isopropyl, butyl or isobutyl group; the methyl and ethyl groups are preferred.

Where $R^6$ represents a $C_2$-$C_4$ alkanoyl group, this may be a straight or branched chain group, for example an acetyl, propionyl, butyryl or isobutyryl group, preferably an acetyl group.

Where $R^3$ and $R^4$ together represent the aforementioned polymethylene group, this may be a trimethylene, tetramethylene or pentamethylene group, preferably a tetramethylene or pentamethylene group.

In the compounds of formula (I), we prefer those compounds in which $R^1$ represents the aforementioned aminoalkylamino group or bis(aminoalkyl)amino group in which the symbols $R^5$ are the same or different and each represents a hydrogen atom, a methyl group or an ethyl group and $\underline{n}$ is 2, or the aforementioned heterocyclic group in which

*n* is 2 and $R^6$ represents a hydrogen atom, a methyl group, an ethyl group, a formyl group or an acetyl group (more preferably a hydrogen atom, a methyl group or a formyl group) or the aforementioned tetrahydro-1,4-thiazin-4-yl group.

Preferred definitions of the group represented by $R^2$ are an amino group, a group of formula $-NHR^5$ (in which $R^5$ represents a hydrogen atom or a $C_1$-$C_4$ alkyl group), a 2-hydroxyethylamino group, a bis(2-hydroxyethyl)amino group or a 2-aminoethylamino group, more preferably an amino group, a methylamino group, an ethylamino group, a 2-hydroxyethylamino group, a bis(2-hydroxyethyl)amino group or a 2-aminoethylamino group.

Where $R^3$ and $R^4$ represent separate groups or atoms, we prefer that one of $R^3$ and $R^4$ should represent a hydrogen atom and that the other should represent a hydrogen atom or a $C_1$-$C_3$ alkyl group. More preferably, $R^3$ represents a hydrogen atom and $R^4$ represents a methyl group or an ethyl group, or $R^3$ represents a methyl group and $R^4$ represents a hydrogen atom, or $R^3$ and $R^4$ both represent hydrogen atoms, most preferably $R^3$ and $R^4$ both represent hydrogen atoms. Alternatively, $R^3$ and $R^4$ preferably together

represent a tetramethylene or pentamethylene group.

The compounds of the invention include the pharmaceutically acceptable acid addition salts of the compounds of formula (I). Any acid may be employed, provided that it does not increase the toxicity of the salt over that of the basic compound or does not do so to an unacceptable extent. Examples of suitable acids include such mineral acids as hydrochloric acid, hydrobromic acid, hydroiodic acid, phosphoric acid, metaphosphoric acid, nitric acid or sulphuric acid, and such organic acids as acetic acid, oxalic acid, tartaric acid, citric acid, benzoic acid, glycolic acid, gluconic acid, glucuronic acid, succinic acid, maleic acid, fumaric acid, malic acid or levulinic acid.

Representative examples of the compounds of formula (I) are given in the following list. The compounds are hereinafter, where appropriate, identified by the numbers assigned to them in this list.

1. 4-Amino-6-ethyl-2-(tetrahydro-1,4-thiazin-4-yl)-thieno[2,3-d]pyrimidine

2. 6-Ethyl-4-methylamino-2-(tetrahydro-1,4-thiazin-4-yl)-thieno[2,3-d]pyrimidine

3. 4-Amino-5-methyl-2-(1-piperazinyl)thieno[2,3-d]-pyrimidine

4. 4-Amino-5-ethyl-2-(1-piperazinyl)thieno[2,3-d]-pyrimidine

5. 4-Amino-2-(1-piperazinyl)-5-propylthieno[2,3-d]-pyrimidine

6. 4-Amino-5-isopropyl-2-(1-piperazinyl)thieno[2,3-d]-pyrimidine

7. 4-Amino-6-methyl-2-(1-piperazinyl)thieno[2,3-d]-pyrimidine

8. 4-Amino-6-ethyl-2-(1-piperazinyl)thieno[2,3-d]-pyrimidine

9. 4-Amino-2-(1-piperazinyl)-6-propylthieno[2,3-d]-pyrimidine

10. 4-Amino-6-butyl-2-(1-piperazinyl)thieno[2,3-d]-pyrimidine

11. 4-Amino-5,6-dimethyl-2-(1-piperazinyl)thieno[2,3-d]-pyrimidine

12. 4-Amino-6-ethyl-5-methyl-2-(1-piperazinyl)thieno[2,3-d]-pyrimidine

13. 4-Amino-5-ethyl-6-methyl-2-(1-piperazinyl)thieno[2,3-d]-pyrimidine

14. 4-Amino-5,6-diethyl-2-(1-piperazinyl)thieno[2,3-d]-pyrimidine

15. 4-Amino-6-butyl-5-ethyl-2-(1-piperazinyl)thieno[2,3-d]-pyrimidine

16. 5-Methyl-4-methylamino-2-(1-piperazinyl)thieno[2,3-d]-pyrimidine

17. 5-Ethyl-4-methylamino-2-(1-piperazinyl)thieno[2,3-d]-pyrimidine

18. 4-Methylamino-2-(1-piperazinyl)-5-propylthieno[2,3-d]-pyrimidine

19. 5-Isopropyl-4-methylamino-2-(1-piperazinyl)thieno-[2,3-d]pyrimidine

20. 6-Methyl-4-methylamino-2-(1-piperazinyl)thieno[2,3-d]-pyrimidine

21. 6-Ethyl-4-methylamino-2-(1-piperazinyl)thieno[2,3-d]-pyrimidine

22. 4-Methylamino-2-(1-piperazinyl)-6-propylthieno[2,3-d]-pyrimidine

23. 6-Butyl-4-methylamino-2-(1-piperazinyl)thieno[2,3-d]-pyrimidine

24. 5,6-Dimethyl-4-methylamino-2-(1-piperazinyl)thieno-[2,3-d]pyrimidine

25. 6-Ethyl-5-methyl-4-methylamino-2-(1-piperazinyl)thieno-[2,3-d]pyrimidine

26. 6-Isopropyl-5-methyl-4-methylamino-2-(1-piperazinyl)-thieno[2,3-d]pyrimidine

27. 5-Ethyl-6-methyl-4-methylamino-2-(1-piperazinyl)thieno-[2,3-d]pyrimidine

28. 5,6-Diethyl-4-methylamino-2-(1-piperazinyl)thieno[2,3-d]-pyrimidine

29. 6-Butyl-5-ethyl-4-methylamino-2-(1-piperazinyl)thieno-[2,3-d]pyrimidine

30. 4-Dimethylamino-5-methyl-2-(1-piperazinyl)thieno[2,3-d]-pyrimidine

31. 4-Dimethylamino-6-ethyl-2-(1-piperazinyl)thieno[2,3-d]-pyrimidine

32. 4-Dimethylamino-5-ethyl-6-methyl-2-(1-piperazinyl)-thieno[2,3-d]pyrimidine

33. 4-Ethylamino-5-methyl-2-(1-piperazinyl)thieno[2,3-d]-pyrimidine

34. 5-Ethyl-4-ethylamino-2-(1-piperazinyl)thieno[2,3-d]-pyrimidine

35. 4-Ethylamino-6-methyl-2-(1-piperazinyl)thieno[2,3-d]-pyrimidine

36. 6-Ethyl-4-ethylamino-2-(1-piperazinyl)thieno[2,3-d]-pyrimidine

37. 5-Ethyl-4-ethylamino-6-methyl-2-(1-piperazinyl)thieno-[2,3-d]pyrimidine

38. 4-Diethylamino-5-methyl-2-(1-piperazinyl)thieno[2,3-d]-pyrimidine

39. 4-Diethylamino-6-ethyl-2-(1-piperazinyl)thieno[2,3-d]-pyrimidine

40. 6-Ethyl-2-(1-piperazinyl)-4-propylaminothieno[2,3-d]-pyrimidine

41. 4-Dipropylamino-5-methyl-2-(1-piperazinyl)thieno[2,3-d]-pyrimidine

42. 4-Isopropylamino-5-methyl-2-(1-piperazinyl)thieno-[2,3-d]pyrimidine

43. 6-Ethyl-4-isopropylamino-2-(1-piperazinyl)thieno[2,3-d]-pyrimidine

44. 5-Ethyl-4-isopropylamino-6-methyl-2-(1-piperazinyl)-thieno[2,3-d]pyrimidine

45. 4-Diisopropylamino-6-ethyl-2-(1-piperazinyl)thieno-[2,3-d]pyrimidine

46. 4-Butylamino-6-ethyl-2-(1-piperazinyl)thieno[2,3-d]-pyrimidine

47. 4-(2-Hydroxyethylamino)-5-methyl-2-(1-piperazinyl)-thieno[2,3-d]pyrimidine

48. 4-Amino-2-(1-piperazinyl)thieno[2,3-d]pyrimidine

49. 4-Methylamino-2-(1-piperazinyl)thieno[2,3-d]pyrimidine

50. 4-Ethylamino-2-(1-piperazinyl)thieno[2,3-d]pyrimidine

51. 5-Ethyl-4-(2-hydroxyethylamino)-2-(1-piperazinyl)-thieno[2,3-d]pyrimidine

52. 4-(2-Hydroxyethylamino)-5-isopropyl-2-(1-piperazinyl)-thieno[2,3-d]pyrimidine

53. 4-(2-Hydroxyethylamino)-6-methyl-2-(1-piperazinyl)-thieno[2,3-d]pyrimidine

54. 6-Ethyl-4-(2-hydroxyethylamino)-2-(1-piperazinyl)-thieno[2,3-d]pyrimidine

55. 4-(2-Hydroxyethylamino)-2-(1-piperazinyl)-6-propylthieno-[2,3-d]pyrimidine

56. 6-Ethyl-4-(2-hydroxyethylamino)-5-methyl-2-(1-piperazinyl)-thieno[2,3-d]pyrimidine

57. 5-Ethyl-4-(2-hydroxyethylamino)-6-methyl-2-(1-piperazinyl)-thieno[2,3-d]pyrimidine

58. 4-Bis(2-hydroxyethyl)amino-5-methyl-2-(1-piperazinyl)-thieno[2,3-d]pyrimidine

59. 4-Bis(2-hydroxyethyl)amino-5-ethyl-2-(1-piperazinyl)-thieno[2,3-d]pyrimidine

60. 4-Bis(2-hydroxyethyl)amino-5-isopropyl-2-(1-piperazinyl)-thieno[2,3-d]pyrimidine

61. 4-Bis(2-hydroxyethyl)amino-6-methyl-2-(1-piperazinyl)-thieno[2,3-d]pyrimidine

62. 4-Bis(2-hydroxyethyl)amino-6-ethyl-2-(1-piperazinyl)-thieno[2,3-d]pyrimidine

63. 4-Bis(2-hydroxyethyl)amino-6-ethyl-5-methyl-2-(1-piperazinyl)thieno[2,3-d]pyrimidine

64. 4-Bis(2-hydroxyethyl)amino-5-ethyl-6-methyl-2-(1-piperazinyl)thieno[2,3-d]pyrimidine

65. 4-(2-Aminoethylamino)-5-methyl-2-(1-piperazinyl)-thieno[2,3-d]pyrimidine

66. 4-(2-Aminoethylamino)-6-ethyl-2-(1-piperazinyl)-thieno[2,3-d]pyrimidine

67. 6-Ethyl-4-(2-methylaminoethylamino)-2-(1-piperazinyl)-thieno[2,3-d]pyrimidine

68. 4-[2-(Ethylamino)ethylamino]-5-methyl-2-(1-piperazinyl)-thieno[2,3-d]pyrimidine

69. 4-[2-(Diethylamino)ethylamino]-5-methyl-2-(1-piperazinyl)-thieno[2,3-d]pyrimidine

70. 4-[2-(Diethylamino)ethylamino]-6-ethyl-2-(1-piperazinyl)-thieno[2,3-d]pyrimidine

71. 4-Bis(2-aminoethyl)amino-6-ethyl-2-(1-piperazinyl)-thieno[2,3-d]pyrimidine

72. 4-Amino-5-methyl-2-(4-methyl-1-piperazinyl)thieno-[2,3-d]pyrimidine

73. 4-Amino-6-ethyl-2-(4-methyl-1-piperazinyl)thieno-[2,3-d]pyrimidine

74. 6-Ethyl-4-(2-hydroxyethylamino)-2-(4-methyl-1-piperazinyl)-thieno[2,3-d]pyrimidine

75. 4-Bis(2-hydroxyethyl)amino-5-methyl-2-(4-methyl-1-piperazinyl)thieno[2,3-d]pyrimidine

76. 4-Amino-6-ethyl-2-(4-ethyl-1-piperazinyl)thieno-[2,3-d]pyrimidine

77. 2-(4-Ethyl-1-piperazinyl)-4-(2-hydroxyethylamino)-5-methylthieno[2,3-d]pyrimidine

78. 4-Amino-6-ethyl-2-(4-propyl-1-piperazinyl)thieno-[2,3-d]pyrimidine

79. 4-Amino-2-(4-formyl-1-piperazinyl)-5-methylthieno-[2,3-d]pyrimidine

80. 4-Amino-5-ethyl-2-(4-formyl-1-piperazinyl)thieno-[2,3-d]pyrimidine

81. 4-Amino-2-(4-formyl-1-piperazinyl)-5-propylthieno-[2,3-d]pyrimidine

82. 4-Amino-2-(4-formyl-1-piperazinyl)-5-isopropylthieno-[2,3-d]pyrimidine

83. 4-Amino-2-(4-formyl-1-piperazinyl)-6-methylthieno-[2,3-d]pyrimidine

84. 4-Amino-6-ethyl-2-(4-formyl-1-piperazinyl)thieno-[2,3-d]pyrimidine

85. 4-Amino-2-(4-formyl-1-piperazinyl)-6-propylthieno-[2,3-d]pyrimidine

86. 4-Amino-6-butyl-2-(4-formyl-1-piperazinyl)thieno-[2,3-d]pyrimidine

87. 4-Amino-2-(4-formyl-1-piperazinyl)-5,6-dimethylthieno-[2,3-d]pyrimidine

88. 4-Amino-6-ethyl-2-(4-formyl-1-piperazinyl)-5-methyl-thieno[2,3-d]pyrimidine

89. 4-Amino-5-ethyl-2-(4-formyl-1-piperazinyl)-6-methylthieno-[2,3-d]pyrimidine

90. 4-Amino-5,6-diethyl-2-(4-formyl-1-piperazinyl)thieno-[2,3-d]pyrimidine

91. 4-Amino-6-butyl-5-ethyl-2-(4-formyl-1-piperazinyl)thieno-[2,3-d]pyrimidine

92. 2-(4-Formyl-1-piperazinyl)-5-methyl-4-methylaminothieno-[2,3-d]pyrimidine

93. 5-Ethyl-2-(4-formyl-1-piperazinyl)-4-methylaminothieno-[2,3-d]pyrimidine

94. 2-(4-Formyl-1-piperazinyl)-4-methylamino-5-propylthieno[2,3-d]pyrimidine

95. 2-(4-Formyl-1-piperazinyl)-5-methyl-4-methylaminothieno-[2,3-d]pyrimidine

96. 5-Ethyl-2-(4-formyl-1-piperazinyl)-4-methylaminothieno-[2,3-d]pyrimidine

97. 2-(4-Formyl-1-piperazinyl)-4-methylamino-5-propylthieno-[2,3-d]pyrimidine

98. 2-(4-Formyl-1-piperazinyl)-6-methyl-4-methylaminothieno-[2,3-d]pyrimidine

99. 6-Ethyl-2-(4-formyl-1-piperazinyl)-4-methylaminothieno-[2,3-d]pyrimidine

100. 2-(4-Formyl-1-piperazinyl)-4-methylamino-6-propylthieno-[2,3-d]pyrimidine

101. 6-Butyl-2-(4-formyl-1-piperazinyl)-4-methylaminothieno-[2,3-d]pyrimidine

102. 2-(4-Formyl-1-piperazinyl)-5,6-dimethyl-4-methylaminothieno-[2,3-d]pyrimidine

103. 6-Ethyl-2-(4-formyl-1-piperazinyl)-5-methyl-4-methylaminothieno[2,3-d]pyrimidine

104. 2-(4-Formyl-1-piperazinyl)-6-isopropyl-5-methyl-4-methylaminothieno[2,3-d]pyrimidine

105. 5-Ethyl-2-(4-formyl-1-piperazinyl)-6-methyl-4-methylaminothieno[2,3-d]pyrimidine

106. 5,6-Diethyl-2-(4-formyl-1-piperazinyl)-4-methylaminothieno[2,3-d]pyrimidine

107. 6-Butyl-5-ethyl-2-(4-formyl-1-piperazinyl)-4-methylaminothieno[2,3-d]pyrimidine

108. 4-Dimethylamino-2-(4-formyl-1-piperazinyl)-5-methylthieno-[2,3-d]pyrimidine

109. 4-Dimethylamino-6-ethyl-2-(4-formyl-1-piperazinyl)-thieno[2,3-d]pyrimidine

110. 4-Dimethylamino-5-ethyl-2-(4-formyl-1-piperazinyl)-6-methylthieno[2,3-d]pyrimidine

111. 4-Ethylamino-2-(4-formyl-1-piperazinyl)-5-methylthieno-[2,3-d]pyrimidine

112. 5-Ethyl-4-ethylamino-2-(4-formyl-1-piperazinyl)-thieno[2,3-d]pyrimidine

113. 4-Ethylamino-2-(4-formyl-1-piperazinyl)-6-methylthieno-[2,3-d]pyrimidine

114. 6-Ethyl-4-ethylamino-2-(4-formyl-1-piperazinyl)thieno-[2,3-d]pyrimidine

115. 5-Ethyl-4-ethylamino-2-(4-formyl-1-piperazinyl)-6-methylthieno[2,3-d]pyrimidine

116. 4-Diethylamino-2-(4-formyl-1-piperazinyl)-5-methyl-thieno[2,3-d]pyrimidine

117. 4-Diethylamino-6-ethyl-2-(4-formyl-1-piperazinyl)-thieno[2,3-d]pyrimidine

118. 6-Ethyl-2-(4-formyl-1-piperazinyl)-4-propylamino-thieno[2,3-d]pyrimidine

119. 4-Dipropylamino-2-(4-formyl-1-piperazinyl)-5-methylthieno[2,3-d]pyrimidine

120. 2-(4-Formyl-1-piperazinyl)-4-isopropylamino-5-methylthieno[2,3-d]pyrimidine

121. 6-Ethyl-2-(4-formyl-1-piperazinyl)-4-isopropylamino-thieno[2,3-d]pyrimidine

122. 5-Ethyl-2-(4-formyl-1-piperazinyl)-4-isopropyl-amino-6-methylthieno[2,3-d]pyrimidine

123. 4-Diisopropylamino-6-ethyl-2-(4-formyl-1-piperazinyl)-thieno[2,3-d]pyrimidine

124. 4-Butylamino-6-ethyl-2-(4-formyl-1-piperazinyl)thieno-[2,3-d]pyrimidine

125. 2-(4-Formyl-1-piperazinyl)-4-(2-hydroxyethylamino)-5-methylthieno[2,3-d]pyrimidine

126. 5-Ethyl-2-(4-formyl-1-piperazinyl)-4-(2-hydroxyethylamino)thieno[2,3-d]pyrimidine

127. 2-(4-Formyl-1-piperazinyl)-4-(2-hydroxyethylamino)-5-isopropylthieno[2,3-d]pyrimidine

128. 2-(4-Formyl-1-piperazinyl)-4-(2-hydroxyethylamino)-6-methylthieno[2,3-d]pyrimidine

129. 6-Ethyl-2-(4-formyl-1-piperazinyl)-4-(2-hydroxyethylamino)thieno[2,3-d]pyrimidine

130. 2-(4-Formyl-1-piperazinyl)-4-(2-hydroxyethylamino)-6-propylthieno[2,3-d]pyrimidine

131. 6-Ethyl-2-(4-formyl-1-piperazinyl)-4-(2-hydroxyethylamino)-5-methylthieno[2,3-d]pyrimidine

132. 5-Ethyl-2-(4-formyl-1-piperazinyl)-4-(2-hydroxyethylamino)-6-methylthieno[2,3-d]pyrimidine

133. 4-Bis(2-hydroxyethyl)amino-2-(4-formyl-1-piperazinyl)-5-methylthieno[2,3-d]pyrimidine

134. 4-Bis(2-hydroxyethyl)amino-5-ethyl-2-(4-formyl-1-piperazinyl)thieno[2,3-d]pyrimidine

135. 4-Bis(2-hydroxyethyl)amino-2-(4-formyl-1-piperazinyl)-5-isopropylthieno[2,3-d]pyrimidine

136. 4-Bis(2-hydroxyethyl)amino-2-(4-formyl-1-piperazinyl)-6-methylthieno[2,3-d]pyrimidine

137. 4-Bis(2-hydroxyethyl)amino-6-ethyl-2-(4-formyl-1-piperazinyl)thieno[2,3-d]pyrimidine

138. 4-Bis(2-hydroxyethyl)amino-6-ethyl-2-(4-formyl-1-piperazinyl)-5-methylthieno[2,3-d]pyrimidine

139. 4-Bis(2-hydroxyethyl)amino-5-ethyl-2-(4-formyl-1-piperazinyl)-6-methylthieno[2,3-d]pyrimidine

140. 4-(2-Aminoethylamino)-2-(4-formyl-1-piperazinyl)-5-methylthieno[2,3-d]pyrimidine

141. 4-(2-Aminoethylamino)-6-ethyl-2-(4-formyl-1-piperazinyl)thieno[2,3-d]pyrimidine

142. 6-Ethyl-2-(4-formyl-1-piperazinyl)-4-(2-methylamino-ethylamino)thieno[2,3-d]pyrimidine

143. 4-[2-(Ethylamino)ethylamino]-2-(4-formyl-1-piperazinyl)-5-methylthieno[2,3-d]pyrimidine

144. 4-[2-(Diethylamino)ethylamino]-2-(4-formyl-1-piperazinyl)-5-methylthieno[2,3-d]pyrimidine

145. 4-[2-(Diethylamino)ethylamino]-6-ethyl-2-(4-formyl-1-piperazinyl)thieno[2,3-d]pyrimidine

146. 4-Bis(2-aminoethyl)amino-6-ethyl-2-(4-formyl-1-piperazinyl)thieno[2,3-d]pyrimidine

147. 2-(4-Acetyl-1-piperazinyl)-4-amino-5-methylthieno-[2,3-d]pyrimidine

148. 2-(4-Acetyl-1-piperazinyl)-4-amino-5-ethylthieno-[2,3-d]pyrimidine

149. 2-(4-Acetyl-1-piperazinyl)-4-amino-5-isopropylthieno-[2,3-d]pyrimidine

150. 2-(4-Acetyl-1-piperazinyl)-4-amino-6-methylthieno-[2,3-d]pyrimidine

151. 2-(4-Acetyl-1-piperazinyl)-4-amino-6-ethylthieno-[2,3-d]pyrimidine

152. 2-(4-Acetyl-1-piperazinyl)-4-amino-6-ethyl-5-methylthieno[2,3-d]pyrimidine

153. 2-(4-Acetyl-1-piperazinyl)-4-(2-hydroxyethylamino)-5-methylthieno[2,3-d]pyrimidine

154. 2-(4-Acetyl-1-piperazinyl)-6-ethyl-4-(2-hydroxyethylamino)thieno[2,3-d]pyrimidine

155. 2-(4-Acetyl-1-piperazinyl)-5-ethyl-4-(2-hydroxyethylamino)-6-methylthieno[2,3-d]pyrimidine

156. 2-(4-Acetyl-1-piperazinyl)-4-bis(2-hydroxyethyl)-amino-6-ethylthieno[2,3-d]pyrimidine

157. 2-(4-Acetyl-1-piperazinyl)-4-bis(2-hydroxyethyl)-amino-6-ethyl-5-methylthieno[2,3-d]pyrimidine

158. 4-Amino-6-ethyl-2-(4-propionyl-1-piperazinyl)thieno-[2,3-d]pyrimidine

159. 4-Amino-2-(4-butyryl-1-piperazinyl)-6-ethylthieno-[2,3-d]pyrimidine

160. 4-Amino-6-ethyl-2-(perhydro-1,4-diazepin-1-yl)thieno-[2,3-d]pyrimidine

161. 4-Amino-6-ethyl-5-methyl-2-(perhydro-1,4-diazepin-1-yl)thieno[2,3-d]pyrimidine

162. 4-Amino-5-ethyl-6-methyl-2-(perhydro-1,4-diazepin-1-yl)thieno[2,3-d]pyrimidine

163. 6-Ethyl-4-(2-hydroxyethylamino)-2-(perhydro-1,4-diazepin-1-yl)thieno[2,3-d]pyrimidine

164. 4-Bis(2-hydroxyethyl)amino-5-methyl-2-(perhydro-1,4-diazepin-1-yl)thieno[2,3-d]pyrimidine

165. 4-Amino-6-ethyl-2-(4-formyl-perhydro-1,4-diazepin-1-yl)thieno[2,3-d]pyrimidine

166. 4-Amino-2-(4-formyl-perhydro-1,4-diazepin-1-yl)-5-methylthieno[2,3-d]pyrimidine

167. 4-Amino-2-(2-aminoethylamino)-6-ethylthieno[2,3-d]-pyrimidine

168. 4-Amino-2-[2-(ethylamino)ethylamino]-5-methylthieno-[2,3-d]pyrimidine

169. 4-Amino-2-[2-(diethylamino)ethylamino]-6-ethylthieno-[2,3-d]pyrimidine

170. 4-Amino-2-[2-(diethylamino)ethylamino]-5-methylthieno-[2,3-d]pyrimidine

171. 4-(2-Aminoethylamino)-2-[2-(diethylamino)ethylamino]-6-ethylthieno[2,3-d]pyrimidine

172. 4-Amino-2-bis[2-(diethylamino)ethyl]amino-5-methyl-thieno[2,3-d]pyrimidine

173. 4-(2-Aminoethylamino)-2-bis[2-(diethylamino)ethyl]-amino-6-ethylthieno[2,3-d]pyrimidine

174. 4-Amino-2-[N-methyl-N-(2-methylaminoethyl)amino]thieno-[2,3-d]pyrimidine

175. 2-[N-Methyl-N-(2-methylaminoethyl)amino]-4-methylamino-thieno[2,3-d]pyrimidine

176. 4-Amino-2-[N-ethyl-N-(2-ethylaminoethyl)amino]-thieno[2,3-d]pyrimidine

177. 2-[N-Ethyl-N-(2-ethylaminoethyl)amino]-4-methyl-aminothieno[2,3-d]pyrimidine

178. 4-Amino-2-(tetrahydro-1,4-thiazin-4-yl)-5,6-trimethylenethieno[2,3-d]pyrimidine

179. 4-Methylamino-2-(tetrahydro-1,4-thiazin-4-yl)-5,6-trimethylenethieno[2,3-d]pyrimidine

180. 4-Amino-2-(tetrahydro-1,4-thiazin-4-yl)-5,6-tetramethylenethieno[2,3-d]pyrimidine

181. 4-Methylamino-2-(tetrahydro-1,4-thiazin-4-yl)-5,6-tetramethylenethieno[2,3-d]pyrimidine

182. 4-Amino-2-(tetrahydro-1,4-thiazin-4-yl)-5,6-pentamethylenethieno[2,3-d]pyrimidine

183. 4-Methylamino-2-(tetrahydro-1,4-thiazin-4-yl)-5,6-pentamethylenethieno[2,3-d]pyrimidine

184. 4-Amino-2-(1-piperazinyl)-5,6-trimethylenethieno-[2,3-d]pyrimidine

185. 4-Amino-2-(1-piperazinyl)-5,6-tetramethylenethieno-[2,3-d]pyrimidine

186. 4-Amino-2-(1-piperazinyl)-5,6-pentamethylenethieno-[2,3-d]pyrimidine

187. 4-Methylamino-2-(1-piperazinyl)-5,6-trimethylenethieno[2,3-d]pyrimidine

188. 4-Methylamino-2-(1-piperazinyl)-5,6-tetramethylenethieno[2,3-d]pyrimidine

189. 4-Methylamino-2-(1-piperazinyl)-5,6-pentamethylenethieno[2,3-d]pyrimidine

190. 4-Ethylamino-2-(1-piperazinyl)-5,6-trimethylenethieno-[2,3-d]pyrimidine

191. 4-Ethylamino-2-(1-piperazinyl)-5,6-tetramethylenethieno[2,3-d]pyrimidine

192. 4-Ethylamino-2-(1-piperazinyl)-5,6-pentamethylene-thieno[2,3-d]pyrimidine

193. 4-(2-Hydroxyethylamino)-2-(1-piperazinyl)-5,6-trimethylenethieno[2,3-d]pyrimidine

194. 4-(2-Hydroxyethylamino)-2-(1-piperazinyl)-5,6-tetramethylenethieno[2,3-d]pyrimidine

195. 4-(2-Hydroxyethylamino)-2-(1-piperazinyl)-5,6-pentamethylenethieno[2,3-d]pyrimidine

196. 4-Bis(2-hydroxyethyl)amino-2-(1-piperazinyl)-5,6-trimethylenethieno[2,3-d]pyrimidine

197. 4-Bis(2-hydroxyethyl)amino-2-(1-piperazinyl)-5,6-tetramethylenethieno[2,3-d]pyrimidine

198. 4-Bis(2-hydroxyethyl)amino-2-(1-piperazinyl)-5,6-pentamethylenethieno[2,3-d]pyrimidine

199. 4-Amino-2-(2-aminoethylamino)-5,6-trimethylenethieno-[2,3-d]pyrimidine

200. 4-Amino-2-(2-aminoethylamino)-5,6-tetramethylenethieno-[2,3-d]pyrimidine

201. 4-Amino-2-(2-aminoethylamino)-5,6-pentamethylenethieno-[2,3-d]pyrimidine

202. 4-Amino-2-(4-methyl-1-piperazinyl)-5,6-trimethylene-thieno[2,3-d]pyrimidine

203. 4-Amino-2-(4-methyl-1-piperazinyl)-5,6-tetramethylene-thieno[2,3-d]pyrimidine

204. 4-Amino-2-(4-methyl-1-piperazinyl)-5,6-pentamethylene-thieno[2,3-d]pyrimidine

205. 4-(2-Hydroxyethylamino)-2-(4-methyl-1-piperazinyl)-5,6-tetramethylenethieno[2,3-d]pyrimidine

206. 4-Bis(2-hydroxyethyl)amino-2-(4-methyl-1-piperazinyl)-5,6-tetramethylenethieno[2,3-d]pyrimidine

207. 4-Amino-2-(4-formyl-1-piperazinyl)-5,6-trimethylene-thieno[2,3-d]pyrimidine

208. 4-Amino-2-(4-formyl-1-piperazinyl)-5,6-tetramethylene-thieno[2,3-d]pyrimidine

209. 4-Amino-2-(4-formyl-1-piperazinyl)-5,6-pentamethylene-thieno[2,3-d]pyrimidine

210. 2-(4-Formyl-1-piperazinyl)-4-methylamino-5,6-trimethylenethieno[2,3-d]pyrimidine

211. 2-(4-Formyl-1-piperazinyl)-4-methylamino-5,6-tetramethylenethieno[2,3-d]pyrimidine

212. 2-(4-Formyl-1-piperazinyl)-4-methylamino-5,6-pentamethylenethieno[2,3-d]pyrimidine

213. 4-Ethylamino-2-(4-formyl-1-piperazinyl)-5,6-tetramethylenethieno[2,3-d]pyrimidine

214. 2-(4-Formyl-1-piperazinyl)-4-(2-hydroxyethylamino)-5,6-trimethylenethieno[2,3-d]pyrimidine

215. 2-(4-Formyl-1-piperazinyl)-4-(2-hydroxyethylamino)-5,6-tetramethylenethieno[2,3-d]pyrimidine

216. 2-(4-Formyl-1-piperazinyl)-4-(2-hydroxyethylamino)-5,6-pentamethylenethieno[2,3-d]pyrimidine

217. 4-Bis(2-hydroxyethyl)amino-2-(4-formyl-1-piperazinyl)-5,6-trimethylenethieno[2,3-d]pyrimidine

218. 4-Bis(2-hydroxyethyl)amino-2-(4-formyl-1-piperazinyl)-5,6-tetramethylenethieno[2,3-d]pyrimidine

 

219. 4-Bis(2-hydroxyethyl)amino-2-(4-formyl-1-piperazinyl)-5,6-pentamethylenethieno[2,3-d]pyrimidine

220. 2-(4-Acetyl-1-piperazinyl)-4-amino-5,6-trimethylene-thieno[2,3-d]pyrimidine

221. 2-(4-Acetyl-1-piperazinyl)-4-amino-5,6-tetramethylene-thieno[2,3-d]pyrimidine

222. 2-(4-Acetyl-1-piperazinyl)-4-amino-5,6-pentamethylene-thieno[2,3-d]pyrimidine

223. 2-(4-Acetyl-1-piperazinyl)-4-(2-hydroxyethylamino)-5,6-trimethylenethieno[2,3-d]pyrimidine

224. 2-(4-Acetyl-1-piperazinyl)-4-(2-hydroxyethylamino)-5,6-tetramethylenethieno[2,3-d]pyrimidine

225. 2-(4-Acetyl-1-piperazinyl)-4-(2-hydroxyethylamino)-5,6-pentamethylenethieno[2,3-d]pyrimidine

226. 4-Amino-5-ethyl-6-methyl-2-(4-methyl-1-piperazinyl)-thieno[2,3-d]pyrimidine

227. 4-Amino-2-[2-(diethylamino)ethylamino]-5-ethyl-6-methylthieno[2,3-d]pyrimidine

228. 4-Amino-2-(4-formyl-1-piperazinyl)-6-isopropylthieno-[2,3-d]pyrimidine

229. 4-Amino-6-isopropyl-2-(1-piperazinyl)thieno[2,3-d]-pyrimidine

230. 6-Isopropyl-4-methylamino-2-(1-piperazinyl)thieno-[2,3-d]pyrimidine

231. 4-Butylamino-5-methyl-2-(1-piperazinyl)thieno[2,3-d]-pyrimidine

232. 4-Methylamino-2-(4-methyl-1-piperazinyl)-5,6-tetramethylenethieno[2,3-d]pyrimidine

Of the compounds listed above, we prefer those identified in the above list as Compounds No. 3, 8, 16, 48, 49, 54, 182 and 197 and their pharmaceutically acceptable acid addition salts, especially the hydrochlorides.

The compounds of the invention may be prepared by reacting a compound of formula (II):

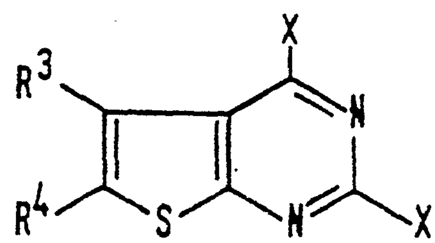

(II)

(in which $R^3$ and $R^4$ are as defined above and X represents a halogen atom, a lower alkylsulphinyl group, an arylsulphinyl group, a lower alkylsulphonyl group or an arylsulphonyl group) with a compound of formula $R^2H$ (in which $R^2$ is as defined above), to give a compound of formula (III):

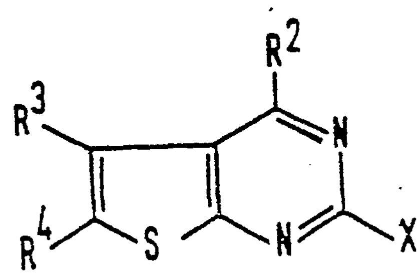

(III)

(in which $R^2$, $R^3$, $R^4$ and X are as defined above), reacting said compound of formula (III) with a compound of formula $R^7H$ (in which $R^7$ represents any one of the groups defined for $R^1$, in which any free amino group is optionally protected), to give a compound of formula (IV):

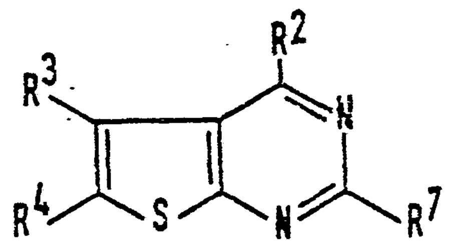

(IV)

(in which $R^2$, $R^3$, $R^4$ and $R^7$ are as defined above), if necessary, removing any amino-protecting groups and optionally acylating a compound in which $R^6$ represents a hydrogen atom to give said compound of formula (I) and, if necessary, salifying said compound.

Where X in the compound of formula (II) employed as the starting material in the preparative process of the invention represents a halogen atom, it is preferably a chlorine, bromine or iodine atom. Compounds of this type can be prepared by the methods disclosed in the Journal of Medicinal Chemistry, 24, 376 (1981) or German Offenlegungsschrift No. 2,200,764.

0082023

When X in the compound of formula (II) represents a lower alkylsulphinyl group, this is preferably a methylsulphinyl, ethylsulphinyl, propylsulphinyl, butylsulphinyl or isobutylsulphinyl group. When X represents an arylsulphinyl group, this is preferably a benzenesulphinyl, p-toluenesulphinyl or naphthylsulphinyl group. When X represents a lower alkylsulphonyl group, this is preferably a methylsulphonyl, ethylsulphonyl, propylsulphonyl, butylsulphonyl or isobutylsulphonyl group. When X represents an arylsulphonyl group, this is preferably a benzenesulphonyl, p-toluenesulphonyl or naphthylsulphonyl group. Compounds of this type can be prepared by alkylating or arylating in a conventional manner a compound of formula (V):

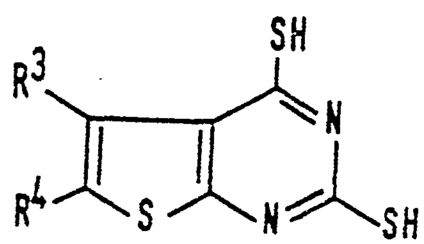

(V)

(in which $R^3$ and $R^4$ are as defined above), which can be prepared by a known method [Synthesis (1972), 268] and then oxidising the resulting compound by conventional means.

Although the groups or atoms represented by the two symbols X in the compound of formula (II) may be the same or different, those compounds in which the two groups or atoms represented by X are identical are more easily obtainable and are therefore preferred.

The first step in the preparative process of the present invention, that is to say the preparation of a compound of formula (III) by reacting a compound of formula (II) with an amine of formula $R^2H$ (in which $R^2$ is as defined above), is preferably effected in the presence of a base and in an inert solvent.

The nature of the base employed in this reaction is not critical and a wide range of organic bases, such as triethylamine, N-methylmorpholine, pyridine, 4-(N,N-dimethylamino)pyridine, N,N-dimethylaniline or 1,8-diazabicyclo-[5.4.0]undecene-7 (DBU), or inorganic bases, such as sodium carbonate, potassium carbonate, sodium hydrogen carbonate or potassium hydrogen carbonate, may be employed. It is also possible to use an excess of the amine compound of formula $R^2H$ as the base. Of these, we particularly prefer to use an organic base, such as triethylamine, N-methylmorpholine, pyridine, N,N-dimethylaniline or DBU, or an excess of the amine compound of formula $R^2H$. The nature of the solvent employed for this reaction

is not critical, provided that it has no adverse effect upon the reaction. Suitable solvents include: aromatic hydrocarbons such as benzene, toluene or xylene; halogenated hydrocarbons, such as methylene chloride or chloroform; esters, such as ethyl acetate or propyl acetate; ethers, such as diethyl ether, tetrahydrofuran or dioxane; alcohols, such as methanol, ethanol, propanol, isopropanol, butanol, isobutanol or isoamyl alcohol; amides, such as dimethylformamide, dimethylacetamide or hexamethylphosphoric triamide; or dimethyl sulphoxide. We particularly prefer to use halogenated hydrocarbons (especially methylene chloride or chloroform), ethers (especially diethyl ether or tetrahydrofuran) or alcohols (especially methanol, ethanol, propanol, isopropanol or isoamyl alcohol).

The reaction temperature may vary over a wide range but is preferably from -10°C to +40°C, more preferably from 0°C to ambient temperature. The time required for the reaction will vary, depending mainly upon the reaction temperature and the nature of the starting materials and base, but it is usually within the range from 1 hour to 30 hours where $R^3$ and $R^4$ represent hydrogen atoms or alkyl groups or from 1 hour to 5 days where $R^3$ and $R^4$ together represent a polymethylene group.

After completion of the reaction, the desired compound of formula (III) can be recovered from the reaction mixture by conventional means. For example, one suitable recovery sequence comprises: adding a water-imiscible organic solvent to the reaction mixture; washing the mixture with water and, if necessary, drying it; and then distilling off the solvent to give the desired compound which can, if necessary, be further purified by conventional means, for example recrystallisation, reprecipitation or chromatography.

The second step in the preparation of the compounds of the invention comprises reacting the compound of formula (III) with a compound of formula $R^7H$ (in which $R^7$ is as defined above) to give a compound of formula (IV). This reaction is preferably effected in the presence of a base and preferably in an inert solvent. Suitable bases and solvents include those illustrated for use in the first step, that is the preparation of the compound of formula (III) from the compound of formula (II).

The temperature employed in this reaction may vary over a wide range, for example from 0°C to 300°C, preferably from 50°C to 200°C. The time required for the reaction will vary, depending mainly upon the reaction

temperature and on the nature of the starting material and base employed, but a period of from 1 to 10 hours will normally suffice.

Compounds of formula (IV) in which $R^2$ and $R^7$ represent the same amino groups can, if desired, be prepared directly by reacting the compound of formula (II) with more than 2 equivalents [per equivalent of said compound of formula (II)] of the amine compound of formula $R^2H$ under the same reaction conditions as are employed for the second reaction step described above.

After completion of the reaction, either the second step alone or the combined first and second steps, the desired compound of formula (IV) can be recovered from the reaction mixture by conventional means. For example, one suitable recovery sequence comprises: adding a water-imiscible organic solvent to the reaction mixture; washing the mixture with water and, if necessary, drying it; and then distilling off the solvent to give the desired compound which may, if necessary, be further purified by conventional means, for example recrystallisation, reprecipitation or chromatography.

The third step in the above reaction sequence is the optional step of de-protecting any protected amino groups; also as part of this step, where a compound has been prepared in which $R^1$ represents the aforementioned heterocyclic amino group and in which $R^6$ represents a hydrogen atom, this may, if desired, be converted to a compound in which $R^6$ represents a formyl group or a $C_2$-$C_4$ alkanoyl group by an acylation reaction. Removal of protecting groups is a reaction well known in the art and the nature of the reaction employed will depend on the nature of the protecting group, as is well known. A wide variety of protecting groups may be employed and their nature is not of importance to the present invention. Generally, the protecting group chosen will be an acyl group selected for its ability to be removed easily by contacting the compound of formula (IV) with an acid (e.g. hydrochloric acid, sulphuric acid, nitric acid, trichloroacetic acid, trifluoroacetic acid or p-toluenesulphonic acid) or an alkali (e.g. sodium hydroxide, potassium hydroxide or lithium hydroxide) in an inert solvent (for example water, an alcohol such as methanol, ethanol or propanol or an aqueous alcohol). The reaction temperature may vary over a wide range, e.g. from ambient temperature to 200°C, preferably from 50°C to 150°C. The time required for the reaction will vary, depending mainly upon the reaction

temperature, the nature of the starting material and the nature of the acid employed, but generally a period of from 10 minutes to 10 hours will suffice.

Conversion of the compound in which $R^6$ represents a hydrogen atom to a compound in which $R^6$ represents a formyl or $C_2$-$C_4$ alkanoyl group can be effected by contacting the compound of formula (I) in which $R^6$ represents a hydrogen atom with a reactive derivative of the carboxylic acid corresponding to the formyl or $C_2$-$C_4$ alkanoyl group, in an inert solvent. Examples of such reactive derivatives include: acid halides, such as acetyl chloride, acetyl bromide, propionyl chloride, propionyl bromide and butyryl chloride; mixed acid anhydrides of monoalkyl carbonates, such as methyl carbonate or ethyl carbonate, or of monoaryl carbonates, such as phenyl carbonate or naphthyl carbonate, with a carboxylic acid, such as acetic acid or propionic acid; and acid anhydrides, such as acetic anhydride or propionic anhydride. We prefer to use acid chlorides or mixed acid anhydrides of monoalkyl carbonates with a carboxylic acid.

The nature of the solvent employed in this reaction is not critical, provided that it has no adverse effect

upon the reaction. Examples of suitable solvents include: aromatic hydrocarbons, such as benzene, toluene or xylene; aliphatic hydrocarbons, such as hexane or heptane; ethers, such as diethyl ether or tetrahydrofuran; halogenated hydrocarbons, such as methylene chloride, chloroform or carbon tetrachloride; ketones, such as acetone or methyl ethyl ketone; and esters, such as ethyl acetate or propyl acetate. Of these, we prefer aromatic hydrocarbons or halogenated hydrocarbons. The reaction temperature is preferably from 0°C to ambient temperature and the time allowed for the reaction is usually from 1 to 20 hours.

Where the reactive derivative of the carboxylic acid employed is an acid chloride or acid anhydride, the reaction is preferably effected in the presence of an acid-binding agent, which may be an organic base, such as triethylamine, N-methylmorpholine, pyridine or 1,8-diazobicyclo[5.4.0]undecene-7 (DBU), or an inorganic base, such as sodium carbonate, potassium carbonate, sodium hydrogen carbonate or potassium hydrogen carbonate. Preferred acid-binding agents are such organic bases as triethylamine, N-methylmorpholine, pyridine or DBU.

After completion of either or both of the above reactions, the desired compound may be recovered from

the reaction mixture by conventional means. For example, one suitable recovery sequence comprises: adding a water-immiscible organic solvent to the reaction mixture; washing the mixture with water and, if necessary, drying it; and then distilling off the solvent to give the desired compound which may, if necessary, be further purified by conventional means, for example recrystallisation, reprecipitation or chromatography.

An alternative method by which the compounds of the invention may be prepared would start with a compound analogous to the compound of formula (II) but in which the group or atom represented by X at the 2-position is replaced by a group $R^7$ (as hereinbefore defined) and the group or atom represented by X at the 4-position is replaced by a group of formula -YH (in which Y represents an oxygen or sulphur atom or a group -NH-); these starting materials may be prepared as described in Libigs Ann. Chem. 699 (1980). These starting materials may be treated in one of the following ways:

(a) The starting material is reacted with a halogenating agent, such as phosphorus oxychloride or phosphorus pentachloride, to give the corresponding 4-halo compound, which is then reacted with a compound of formula $R^2H$ (in which $R^2$ is as defined above), in the presence

of a base, after which, if necessary, the resulting compound is deprotected to give the desired compound of formula (I). Each of the reactions mentioned above, that is to say the reaction with the halogenating agent and the reaction with the compound of formula $R^2H$, is preferably effected at a temperature of from ambient temperature to the reflux temperature of the reaction mixture.

(b) the starting material is alkylated with an alkylating agent (such as a dialkyl sulphate or alkyl halide, e.g. methyl iodide). Where Y represents an oxygen atom or a group -NH-, the resulting compound is reacted with a compound of formula $R^2H$ and then, if necessary, deprotected to give a compound of formula (I). Alternatively, if Y represents a sulphur atom, the product of the alkylation is oxidised, e.g. by m-chloroperbenzoic acid, to give the corresponding 4-alkylsulphinyl or 4-alkylsulphonyl compound, which is reacted with a compound of formula $R^2H$, optionally in the presence of a base, and then, if necessary, the resulting product is deprotected to give the desired compound of formula (I).

The resulting compound may then be separated from the reaction mixture and, if necessary, further purified by conventional means.

The compounds of the invention are useful for the treatment of a variety of disorders. Specifically, many of the compounds of the invention have the ability to inhibit the aggregation of blood platelets and are thus useful for the prophylaxis and therapy of thrombosis. Compounds of the invention in which $R^2$ represents an amino group or a monoalkylamino group and one or both of $R^3$ and $R^4$ represents a hydrogen atom additionally possess hypoglycaemic activity and can be used for the treatment of various hypoglycaemic conditions, such as diabetes mellitus. Compounds in which $R^1$ represents a 1-piperazinyl group also have strong hypoglycaemic activity. Compounds in which $R^1$ represents a tetrahydro-1,4-thiazin-4-yl (or thiomorpholino) group additionally possess remarkable hypotensive activity and can be used for the treatment of hypertension. Certain specific compounds of the invention have also been found to exhibit remarkable diuretic activity and can be used for the treatment of oedema and of hypertension.

The compounds of the invention may be administered orally (for example in the form of tablets, capsules, granules, powders or syrups) or parenterally (in the form of injectable solutions) or they may be administered as suppositories. The dose will vary, depending upon the age and condition of the patient, as well as the disorder which is to be treated and the route of administration, but the adult daily dose is usually from 1 to 1,000 mg, more preferably from 2 to 500 mg, which may be administered as a single dose or in divided doses.

The various activities of certain of the compounds of the invention are demonstrated by the following experiments. In these, the compounds of the invention are identified by the numbers assigned to them in the foregoing list.

Hypoglycaemic activity

In this experiment, the hypoglycaemic activity of various of the compounds of the invention was measured in mice after loading a large quantity of glucose. The test animals were male mice of the ddY strain having a body weight of from 25 to 27 g. Each experiment was

carried out on a group of 6 mice.

Each test compound was administered orally in an amount of 10 mg/kg to the mouse followed, 10 minutes later, by the oral administration of 1.2 g/kg of D-glucose. 30 minutes after administration of the D-glucose, blood was collected from each mouse and the blood sugar concentration was measured using a commercially available kit ("New Blood Sugar Test", a product of Boehringer-Mannheim Yamanouchi Pharmaceutical Co. Ltd,). The inhibitory rate, R, was calculated from the following formula:

$$R = (1 - \frac{B}{A}) \times 100$$

in which A represents the blood sugar concentration of a positive control group and B represents the blood sugar concentration of the group to which the test compound was administered. The results are shown in Table 1. As a control, the experiment was also carried out with: the known hypoglycaemic agent, tolbutamide; 2-(2-aminoethylamino)-4-morpholinothieno[2,3-d]pyrimidine (named "VK-744"), a compound disclosed in German Offenlegungsschrift No. 2,200,764; and 4-isopropylamino-2-piperidinothieno[2,3-d]-pyrimidine (hereinafter referred to as "ICI-154"), a compound disclosed in U.S. Patent Specification No. 4,146,716. The results are shown in Table 1.

TABLE 1

| Compound No. | Inhibitory rate, R% |
|---|---|
| 3 | 32.4 |
| 8 | 34.0 |
| 16 | 43.9 |
| 21 | 20.0 |
| 39 | 19.4 |
| 49 | 32.0 |
| 66 | 32.9 |
| 73 | 34.1 |
| 84 | 39.3 |
| 99 | 22.1 |
| tolbutamide | 2.8 |
| VK-744 | 19.0 |
| ICI-154 | 11.0 |

The results shown in the above Table suggest that the majority of the compounds of the invention have an activity which is at least an order of magnitude greater than that of the known hypoglycaemic agent, tolbutamide. The majority of the compounds of the invention

0082023

tested also appear more effective than VK-744 and all appear more effective than ICI-154, although the hypoglycaemic activity of these compounds has not hitherto been disclosed.

Inhibitory activity on platelet aggregation

The ability of various compounds of the present invention to inhibit platelet aggregation was measured using rabbit blood.

9 volumes of blood collected from male rabbit hearts and 1 volume of a 3.8% w/v aqueous solution of sodium citrate were mixed, placed in a centrifugal separator and subjected to centrifugation under 150 G for 15 minutes to give a platelet-rich-plasma (PRP). 250 µl of this PRP were placed in a cuvette and incubated for 2 minutes with a test compound at the concentration shown in Table 2. To the mixture was then added a collagen solution to a final concentration of 5 µg/ml, to induce aggregation. The aggregation was measured using an aggregometer produced by Rikadenki Co. Ltd. The inhibitory rate R' was calculated from the following formula:

$$R' = (1 - \frac{B'}{A'}) \times 100$$

in which A' represents the maximum aggregation rate when only a solvent was added and B' represents the maximum aggregation rate when the test compound was added.

For comparison, the experiment was also carried out using as the test compound VK-744, of German Offenlegungsschrift No. 2,200,764, and ICI-154 of U.S. Patent Specification No. 4,146,716. The results are shown in Table 2.

TABLE 2

| Compound No. | Concentration (g/ml) | Inhibitory rate, R'% |
|---|---|---|
| 3 | $10^{-5}$ | 82.4 |
| 54 | $10^{-6}$ | 66.1 |
| 62 | $10^{-6}$ | 65.3 |
| 66 | $3 \times 10^{-5}$ | 100 |
| 73 | $3 \times 10^{-5}$ | 100 |
| 197 | $10^{-5}$ | 87 |
| VK-744 | $10^{-4}$ | 100 |
| | $10^{-5}$ | < 50 |
| ICI-154 | $10^{-4}$ | < 50 |

Diuretic Activity

The animals employed in this test were male mice of the ddY strain, each having a body weight of from 26 g to 30 g. The mice were employed in groups of five for each test.

Each test compound was suspended in a 0.3% carboxymethylcellulose saline solution and administered orally in the dose shown in Table 3 to the mouse. For the period of 2 hours after administration, the urine was collected and the urine volume and concentration of sodium ions were measured. These results were compared with results obtained from a control group, to which the carboxymethylcellulose saline solution alone was administered,and the results are reported in Table 3 as the percentage increase in urine volume and sodium ion concentration over that of the control group. The compounds tested were Compound No. 3 of the present invention and hydrochlorothiazide (abbreviated as "HCT").

TABLE 3

| Compound | Dose (mg/kg) | Percent increase in | |
|---|---|---|---|
| | | Urine volume | $Na^+$ conc. |
| No. 3 | 100 | +76 | +51 |
| HCT | 30 | +43 | +93 |

Hypotensive Activity

The animals used in this test were 15 week old male spontaneously hypertensive rats. 3-5 rats were used to test each concentration of each compound.

Each animal was treated as follows. The animal was anaesthetized by the intraperitoneal administration of 50 mg/kg of sodium pentobarbital. A polyethylene cannula was then inserted into the abdominal aorta by the method of Weeks and Jones [J.R. Weeks and J.A. Jones, Proc. Soc. Experimental Biol. Med., 104, 646-648 (1960)]. The other end of the cannula, leading out of the body, was fixed to the neck. One week after the operation, when the animal had recovered from post-operative invasion, this end of the cannula was connected to a system for measuring blood pressure, in order to measure the blood pressure and heart beat directly without the need for any further anaesthesia or restraint. The system for measuring blood pressure employed was an improved version of that originated by Laffan et al. [P.J. Laffan, A. Peterson, S.W. Hitch and C. Jeunelot, Cardiovascular Res., 6, 319-324 (1972)].

The compound under test was suspended at the desired concentration in a 0.3% w/v aqueous solution of carboxymethylcellulose and administered orally in a total

dose of test compound of 100 mg/kg. The blood pressure and heart beat of each animal were observed for 1 hour before administration to give control values and then the animal was given the sample when these values were stable. After administration of the sample, the blood pressure and heart beat of each animal were measured for 24 hours at 15 minute intervals.

The results are shown in the following Table 4, in which the abbreviations used are as follows:

$\Delta_{max}$= maximum change in blood pressure (mmHg) after administration of the compound under test;

$T_{max}$= time (in hours) after administration of the test compound required for the blood pressure to attain its maximum change;

$T_{0.5max}$= time (in hours) after administration of the compound under test for the blood pressure to return to one half of its maximum value.

Table 4

| Compound No. | Δ max (mmHg) | $T_{max}$ (hours) | $T_{0.5max}$ (hours) |
|---|---|---|---|
| 1 | -38 | 0.75 | 8.5 |
| 27 | -36 | 7 | 10 |
| 69 | -29 | 6 | 9 |
| 180 | -21 | 3 | 6 |
| 182 | -51 | 5 | 10 |

The preparation of the compounds of the invention is illustrated by the following Examples, whilst the preparation of certain intermediates used to prepare the compounds of the invention is illustrated in the following Preparations.

PREPARATION 1

4-Amino-2-chloro-6-ethylthieno[2,3-d]pyrimidine

100 ml of tetrahydrofuran were saturated with ammonia, with ice-cooling. To the mixture were added 4.5 g of 2,4-dichloro-6-ethylthieno[2,3-d]pyrimidine, melting at 68-70°C. The mixture was left to stand overnight at room temperature, after which the solvent was distilled off and the crystals which were produced were washed with water and collected by filtration. The crystals were recrystallised from ethanol, giving 3.2 g (yield 78%) of the title compound, melting at 225-226°C.

Elemental Analysis:

Calculated for $C_8H_8N_3SCl$: C, 44.97%; H, 3.77%; N, 19.66%; S, 15.00%; Cl, 16.59%.

Found : C, 44.69%; H, 3.75%; N, 19.37%; S, 15.14%; Cl, 16.71%.

## PREPARATIONS 2 - 20

The procedure of Example 1 was repeated, except that ammonia was replaced by an amine and/or a different 2,4-dichloro-thieno[2,3-d]pyrimidine derivative was employed, to give the following compounds:

Preparation 2: 4-amino-2-chloro-5-methylthieno[2,3-d]pyrimidine, melting at 211-213°C.

Preparation 3: 2-chloro-5-methyl-4-methylaminothieno[2,3-d]-pyrimidine, melting at 205-207°C.

Preparation 4: 2-chloro-6-ethyl-4-methylaminothieno[2,3-d]-pyrimidine, melting at 174-175°C.

Preparation 5: 2-chloro-6-ethyl-4-isopropylaminothieno[2,3-d]-pyrimidine, melting at 150-152°C.

Preparation 6: 2-chloro-4-diethylamino-6-ethylthieno[2,3-d]-pyrimidine, melting at 78-80°C.

Preparation 7: 2-chloro-6-ethyl-4-(2-hydroxyethylamino)thieno-[2,3-d]pyrimidine, melting at 149-151°C.

Preparation 8: 4-bis(2-hydroxyethyl)amino-2-chloro-6-ethylthieno[2,3-d]pyrimidine, melting at 131-133°C.

Preparation 9: 4-(2-acetylaminoethylamino)-2-chloro-6-ethylthieno[2,3-d]pyrimidine, melting at 250-251°C.

Preparation 10: 2-chloro-5-ethyl-6-methyl-4-methylaminothieno-[2,3-d]pyrimidine, melting at 177-179°C.

Preparation 11: 2-chloro-5-ethyl-4-(2-hydroxyethylamino)-6-methylthieno[2,3-d]pyrimidine, melting at 167-169°C.

Preparation 12: 4-amino-2-chloro-5-ethyl-6-methylthieno[2,3-d]-pyrimidine, melting at 215-217°C.

Preparation 13: 2-chloro-4-[2-(diethylamino)ethylamino]-6-ethylthieno[2,3-d]pyrimidine, melting at 161-165°C.

Preparation 14: 4-amino-2-chloro-6-methylthieno[2,3-d]pyrimidine, melting at 258-259°C.

Preparation 15: 2-chloro-6-methyl-4-methylaminothieno[2,3-d]-pyrimidine, melting at 241-242°C.

Preparation 16: 2-chloro-6-ethyl-4-ethylaminothieno[2,3-d]-pyrimidine, melting at 143-144°C.

Preparation 17: 4-amino-2-chloro-6-isopropylthieno[2,3-d]-pyrimidine, melting at 213-215°C.

Preparation 18: 2-chloro-6-isopropyl-4-methylaminothieno-[2,3-d]pyrimidine, melting at 122-124°C.

Preparation 19: 2-chloro-5-isopropyl-4-methylaminothieno-[2,3-d]pyrimidine, melting at 124-126°C.

Preparation 20: 4-amino-2-chloro-5-isopropylthieno[2,3-d]-pyrimidine, melting at 174-176°C.

## PREPARATION 21

### 4-Amino-2-chloro-5,6-tetramethylenethieno[2,3-d]pyrimidine

900 ml of tetrahydrofuran were saturated with ammonia, with ice-cooling. To the mixture were added 30 g of 2,4-dichloro-5,6-tetramethylenethieno[2,3-d]pyrimidine and the mixture was stirred for 4 days at room temperature.

0082023

The solvent was then distilled off and the crystals produced were washed with water and collected by filtration, after which they were recrystallised from tetrahydrofuran, to give the title compound in a yield of 80%, melting at 278-280°C (with decomposition).

PREPARATIONS 22 - 27

The procedure described in Preparation 21 was repeated, but employing different starting materials, to give the following compounds:

Preparation 22: 4-amino-2-chloro-5,6-trimethylenethieno[2,3-d]-pyrimidine, melting at 278-280°C (with decomposition), yield 65%.

Preparation 23: 4-amino-2-chloro-5,6-pentamethylenethieno[2,3-d]-pyrimidine, melting at 255-257°C, yield 55%.

Preparation 24: 2-chloro-4-methylamino-5,6-tetramethylenethieno-[2,3-d]pyrimidine, melting at 194-196°C, yield 92%.

Preparation 25: 2-chloro-4-methylamino-5,6-trimethylenethieno-[2,3-d]pyrimidine, melting at 201-202°C, yield 94%.

Preparation 26: 2-chloro-4-(2-hydroxyethylamino)-5,6-tetramethylenethieno[2,3-d]pyrimidine, melting at 171-172°C, yield 78%.

Preparation 27: 4-bis(2-hydroxyethyl)amino-2-chloro-5,6-tetramethylenethieno[2,3-d]pyrimidine, melting at 122-124°C, yield 78%.

## EXAMPLE 1

4-Amino-6-ethyl-2-(4-methyl-1-piperazinyl)thieno[2,3-d]-pyrimidine, Compound No. 73

0.71 g of 4-amino-2-chloro-6-ethylthieno[2,3-d]-pyrimidine and 0.92 g of 4-methylpiperazine were refluxed with 5 ml of isoamyl alcohol for 5 hours, after which the isoamyl alcohol was distilled off under reduced pressure and the residue was dissolved in chloroform and then washed with water. The chloroform layer was dried over anhydrous magnesium sulphate and then the chloroform was distilled off. The residue was recrystallised from ethyl acetate, to give 0.67 g (yield 68%) of the title compound, melting at 113-118°C.

Infrared Absorption Spectrum (KBr) $\nu_{max} cm^{-1}$:

3390, 3340, 3200, 1650, 1575.

Mass Spectrum m/e:

277 ($M^+$), 207.

## EXAMPLE 2

4-Amino-6-ethyl-2-(4-formyl-1-piperazinyl)thieno[2,3-d]-pyrimidine, Compound No. 84

The procedure described in Example 1 was repeated, except that the 1-methylpiperazine was replaced by 1-formylpiperazine, to give the title compound, melting at 196-198°C, in a yield of 65%.

Infrared Absorption Spectrum (KBr) $\nu_{max} cm^{-1}$:

3400, 3320, 3190, 1655, 1640.

Mass Spectrum m/e:

291 ($M^+$), 233, 207.

## EXAMPLE 3

6-Ethyl-2-(4-formyl-1-piperazinyl)-4-methylaminothieno[2,3-d]-pyrimidine, Compound No. 99

2.28 g of 2-chloro-6-ethyl-4-methylaminothieno[2,3-d]-pyrimidine and 2.4 g of 1-formylpiperazine were refluxed for 4 hours with 25 ml of isoamyl alcohol, after which the isoamyl alcohol was distilled off under reduced pressure and the residue was dissolved in chloroform. The chloroform solution was washed with water and then dried over anhydrous magnesium sulphate, after which the chloroform was distilled off. The residue was then subjected to column chromatography through silica gel eluted with a 2% v/v mixture of ethanol in chloroform. The residue from the eluate was recrystallised from ethyl acetate, to give 1.97 g (yield 64%) of the title compound in the form of colourless needles melting at 174-175°C.

Infrared Absorption Spectrum (KBr) $\nu_{max}cm^{-1}$:

3360, 1668, 1660.

Mass Spectrum m/e:

305 ($M^+$), 221.

## EXAMPLE 4

6-Ethyl-2-(4-formyl-1-piperazinyl)-4-isopropylaminothieno-[2,3-d]pyrimidine, Compound No. 121

The procedure described in Example 3 was repeated,

except that the 2-chloro-6-ethyl-4-methylaminothieno[2,3-d]-pyrimidine was replaced by 1.30 g of 2-chloro-6-ethyl-4-isopropylaminothieno[2,3-d]pyrimidine, to give 0.80 g (yield 47%) of the title compound melting at 209-211°C.

Infrared Absorption Spectrum (KBr) $\nu_{max}$ $cm^{-1}$:

3360, 1668, 1595.

Mass Spectrum m/e:

333 ($M^+$), 249.

EXAMPLE 5

4-Diethylamino-6-ethyl-2-(4-formyl-1-piperazinyl)thieno-[2,3-d]pyrimidine, Compound No. 117

The procedure described in Example 3 was repeated, except that the 2-chloro-6-ethyl-4-methylaminothieno[2,3-d]-pyrimidine was replaced by 1.50 g of 2-chloro-4-diethylamino-6-ethylthieno[2,3-d]pyrimidine, to give 0.96 g (yield 55%) of the title compound, melting at 198-199°C.

Infrared Absorption Spectrum (KBr) $\nu_{max}$ $cm^{-1}$:

1660, 1645.

Mass Spectrum m/e:

347 ($M^+$), 263.

## EXAMPLE 6

6-Ethyl-2-(4-formyl-1-piperazinyl)-4-(2-hydroxyethylamino)thieno-[2,3-d]pyrimidine, Compound No. 129

The procedure described in Example 3 was repeated, except that the 2-chloro-6-ethyl-4-methylaminothieno[2,3-d]-pyrimidine was replaced by 1.00 g of 2-chloro-6-ethyl-4-(2-hydroxyethylamino)thieno[2,3-d]pyrimidine, to give 0.77 g (yield 59%) of the title compound, melting at 180-182°C.

Infrared Absorption Spectrum (KBr) $\nu_{max}$ $cm^{-1}$:

3460, 3320, 3200, 1670, 1610.

Mass Spectrum m/e:

335 ($M^+$), 251.

## EXAMPLE 7

4-Bis(2-hydroxyethyl)amino-6-ethyl-2-(4-formyl-1-piperazinyl)-thieno[2,3-d]pyrimidine, Compound No. 137

The procedure described in Example 3 was repeated, except that the 2-chloro-6-ethyl-4-methylaminothieno[2,3-d]-pyrimidine was replaced by 2.40 g of 4-bis(2-hydroxyethyl)-amino-2-chloro-6-ethylthieno[2,3-d]pyrimidine, to give

1.80 g (yield 60%) of the title compound, melting at 156-158°C.

Infrared Absorption Spectrum (KBr) $\nu_{max}$ $cm^{-1}$:

3360, 3230, 1655.

Mass Spectrum m/e:

379 ($M^+$), 348.

EXAMPLE 8

4-[2-(Diethylamino)ethylamino]-6-ethyl-2-(4-formyl-1-piperazinyl)-thieno[2,3-d]pyrimidine, Compound No. 145

The procedure described in Example 3 was repeated, except that the 2-chloro-6-ethyl-4-methylaminothieno[2,3-d]-pyrimidine was replaced by 2.20 g of 2-chloro-4-[2-(diethylamino)-ethylamino]-6-ethylthieno[2,3-d]pyrimidine, to give 2.47 g (yield 90%) of the title compound as an oily substance.

Mass Spectrum m/e:

390 ($M^+$), 291.

EXAMPLE 9

4-Amino-2-(4-formyl-1-piperazinyl)-5-methylthieno[2,3-d]-pyrimidine, Compound No. 79

The procedure described in Example 3 was repeated, except that the 2-chloro-6-ethyl-4-methylaminothieno[2,3-d]-pyrimidine was replaced by 3.71 g of 4-amino-2-chloro-5-methylthieno-[2,3-d]pyrimidine, to give 2.04 g (yield 46%) of the title compound, melting at 205-207°C.

Infrared Absorption Spectrum (KBr) $\nu_{max}$ $cm^{-1}$:

3510, 3340, 1665, 1620.

Mass Spectrum m/e:

277 ($M^+$), 193.

## EXAMPLE 10

2-(4-Formyl-1-piperazinyl)-5-methyl-4-methylaminothieno[2,3-d]-pyrimidine, Compound No. 92

The procedure described in Example 3 was repeated, except that the 2-chloro-6-ethyl-4-methylaminothieno[2,3-d]-pyrimidine was replaced by 1.50 g of 2-chloro-5-methyl-4-methylaminothieno[2,3-d]pyrimidine, to give 0.88 g (yield 43%) of the title compound, melting at 169-171°C.

Infrared Absorption Spectrum (KBr) $\nu_{max}$ $cm^{-1}$:

3450, 1675, 1580, 1565.

Mass Spectrum m/e:

291 ($M^+$), 207.

EXAMPLE 11

4-Amino-5-methyl-2-(4-methyl-1-piperazinyl)thieno[2,3-d]-pyrimidine, Compound No. 72

2.00 g of 4-amino-2-chloro-5-methylthieno[2,3-d]-pyrimidine and 1.00 g of 1-methylpiperazine were heated in a sealed tube with 5 ml of isoamyl alcohol at 180°C for 5 hours. The isoamyl alcohol was then distilled off under reduced pressure and the residue was dissolved in chloroform and washed with water. The chloroform was distilled off. To the residue was added 10% w/v hydrochloric acid and the mixture was concentrated by evaporation under reduced pressure. The residue was recrystallized from a 1:1 by volume mixture of ethanol and acetone, to give 1.85 g (yield 55.0%) of the title compound in the form of its hydrochloride, melting at 293-295°C (with decomposition).

Infrared Absorption Spectrum (KBr) $\nu_{max}$ $cm^{-1}$:

3490, 3400, 3290, 3150, 1645.

Mass Spectrum m/e:

263 ($M^+$), 193.

EXAMPLE 12

4-Amino-6-ethyl-2-(1-piperazinyl)thieno[2,3-d]pyrimidine, Compound No. 8

A mixture of 1.10 g of 4-amino-6-ethyl-2-(4-formyl-1-piperazinyl)thieno[2,3-d]pyrimidine (Compound No. 84) and 15 ml of 10% w/v hydrochloric acid was heated under reflux for 1 hour and then cooled. The mixture was then neutralized with 10% v/v aqueous ammonia and the resulting crystals were collected by filtration, dried and then recrystallized from ethanol to give the title compound, melting at 165-167°C, in 80% yield.

Infrared Absorption Spectrum (KBr) $\nu_{max}$ $cm^{-1}$:

3360, 3280, 3190, 1630, 1595, 1590, 1570, 1555.

Mass Spectrum m/e:

263 ($M^+$), 221, 207.

EXAMPLE 13

6-Ethyl-4-methylamino-2-(1-piperazinyl)thieno[2,3-d]pyrimidine, Compound No. 21

The procedure described in Example 12 was repeated, except that the Compound No. 84 was replaced by 6-ethyl-2-(4-formyl-1-piperazinyl)-4-methylaminothieno[2,3-d]pyrimidine (Compound No. 99), to give the title compound,

melting at 170-172°C, in 95% yield.

Infrared Absorption Spectrum (KBr) $\nu_{max}$ $cm^{-1}$:

3240, 3150, 1595, 1562.

Mass Spectrum m/e:

277 ($M^+$), 235, 221.

## EXAMPLE 14

6-Ethyl-4-isopropylamino-2-(1-piperazinyl)thieno[2,3-d]pyrimidine, Compound No. 43

The procedure described in Example 12 was repeated, except that the Compound No. 84 was replaced by 6-ethyl-2-(4-formyl-1-piperazinyl)-4-isopropylaminothieno[2,3-d]pyrimidine (Compound No. 121), to give the title compound, melting at 174-176°C, in 50% yield.

Infrared Absorption Spectrum (KBr) $\nu_{max}$ $cm^{-1}$:

3300, 1585, 1560.

Mass Spectrum m/e:

305 ($M^+$), 263, 249.

## EXAMPLE 15

4-Diethylamino-6-ethyl-2-(1-piperazinyl)thieno[2,3-d]pyrimidine, Compound No. 39

The procedure described in Example 12 was repeated, except that the Compound 84 was replaced by 4-diethylamino-6-ethyl-2-(4-formyl-1-piperazinyl)thieno[2,3-d]pyrimidine (Compound No. 117) to give the title compound, melting at 56-58°C, in 68% yield.

Infrared Absorption Spectrum (KBr) $\nu_{max}$ $cm^{-1}$:

3440, 3230, 1540.

Mass Spectrum m/e:

319 ($M^+$), 263.

## EXAMPLE 16

6-Ethyl-4-(2-hydroxyethylamino)-2-(1-piperazinyl)thieno-[2,3-d]pyrimidine, Compound No. 54

The procedure described in Example 12 was repeated, except that the Compound No. 84 was replaced by 6-ethyl-2-(4-formyl-1-piperazinyl)-4-(2-hydroxyethylamino)thieno[2,3-d]-pyrimidine (Compound No. 129), to give the title compound, melting at 174-176°C, in 61% yield.

Infrared Absorption Spectrum (KBr) $\nu_{max}$ $cm^{-1}$:

3330, 1590, 1565.

Mass Spectrum m/e:

307 ($M^+$), 265, 251, 239.

EXAMPLE 17

4-Bis(2-hydroxyethyl)amino-6-ethyl-2-(1-piperazinyl)thieno-[2,3-d]pyrimidine, Compound No.62

The procedure described in Example 12 was repeated, except that the 4-amino-6-ethyl-2-(4-formyl-1-piperazinyl)-thieno[2,3-d]pyrimidine was replaced by 4-bis(2-hydroxyethyl)-amino-6-ethyl-2-(4-formyl-1-piperazinyl)thieno[2,3-d]pyrimidine, to give the title compound, melting at 148-150°C, in a yield of 45%.

Infrared Absorption Spectrum (KBr) $\nu_{max}$ $cm^{-1}$:

3400, 1540, 1530.

Mass Spectrum m/e:

351 ($M^+$), 309, 295.

EXAMPLE 18

4-[2-(Diethylamino)ethylamino]-6-ethyl-2-(1-piperazinyl)thieno-[2,3-d]pyrimidine, Compound No. 70

The procedure described in Example 12 was repeated, except that the 4-amino-6-ethyl-2-(4-formyl-1-piperazinyl)-thieno[2,3-d]pyrimidine was replaced by 4-[2-(diethylamino)-ethylamino]-6-ethyl-2-(4-formyl-1-piperazinyl)thieno[2,3-d]-pyrimidine, to give the title compound, melting at 87-89°C, in a yield of 54%.

Infrared Absorption Spectrum (KBr) $\nu_{max}$ $cm^{-1}$:

3290, 1600, 1565.

Mass Spectrum m/e:

362 ($M^+$), 263.

EXAMPLE 19

4-Amino-5-methyl-2-(1-piperazinyl)thieno[2,3-d]pyrimidine, Compound No. 3

The procedure described in Example 12 was repeated,

except that the 4-amino-6-ethyl-2-(4-formyl-1-piperazinyl)-thieno[2,3-d]pyrimidine was replaced by 4-amino-2-(4-formyl-1-piperazinyl)-5-methylthieno[2,3-d]pyrimidine, to give the title compound, melting at 144-146°C, in a yield of 56%.

Infrared Absorption Spectrum (KBr) $\nu_{max}$ $cm^{-1}$:

3500, 3300, 1638, 1565.

Mass Spectrum m/e:

249 ($M^+$), 207.

EXAMPLE 20

5-Methyl-4-methylamino-2-(1-piperazinyl)thieno[2,3-d]pyrimidine, Compound No. 16

A mixture of 0.35 g of 2-(4-formyl-1-piperazinyl)-5-methyl-4-methylaminothieno[2,3-d]pyrimidine (Compound No. 92) and 20 ml of 10% w/v hydrochloric acid was heated on a water bath for 30 minutes and then cooled, to give the hydrochloride of the title compound, melting at 296-299°C (with decomposition), in a yield of 75%.

Infrared Absorption Spectrum (KBr) $\nu_{max}$ $cm^{-1}$:

3370, 1615, 1585.

Mass Spectrum m/e:

263 ($M^+$), 221, 205.

## EXAMPLE 21

4-(2-Aminoethylamino)-6-ethyl-2-(1-piperazinyl)thieno[2,3-d]-pyrimidine, Compound No. 66

(a) The procedure described in Example 3 was repeated, except that the 2-chloro-6-ethyl-4-methylaminothieno-[2,3-d]pyrimidine was replaced by 4-(2-acetylaminoethylamino)-2-chloro-6-ethylthieno[2,3-d]pyrimidine, to give 4-(2-acetylaminoethylamino)-6-ethyl-2-(4-formyl-1-piperazinyl)thieno-[2,3-d]pyrimidine, melting at 214-216°C, in a yield of 34%.

Infrared Absorption Spectrum (KBr) $\nu_{max}$ $cm^{-1}$:

3340, 3280, 1672, 1655.

Mass Spectrum m/e:

376 ($M^+$), 292.

(b) A mixture of 0.3 g of 4-(2-acetylaminoethylamino)-6-ethyl-2-(4-formyl-1-piperazinyl)thieno[2,3-d]-pyrimidine and 10 ml of 10% w/v hydrochloric acid was heated under reflux for 5 hours. The reaction mixture was then concentrated by evaporation under reduced pressure and the residue was recrystallised from 80% v/v aqueous ethanol, to give the title compound, in the form of its hydrochloride, melting at 286-288°C (with decomposition), in a yield of 66%.

Infrared Absorption Spectrum (KBr) $\nu_{max}$ $cm^{-1}$:

3440, 3200, 1620, 1590, 1570.

Mass Spectrum m/e:

306 ($M^+$), 264, 250, 238, 233.

## EXAMPLE 22

2-(4-Acetyl-1-piperazinyl)-4-amino-5-methylthieno[2,3-d]-pyrimidine, Compound No. 147

To 10 ml of chloroform were added 0.10 g of 4-amino-5-methyl-2-(1-piperazinyl)thieno[2,3-d]pyrimidine

(Compound No. 3) and 0.11 g of triethylamine. The mixture was then ice-cooled, while 0.045 g of acetyl chloride were added, after which the mixture was stirred at room temperature for 16 hours. The mixture was then washed with water and the solvent was distilled off. The resulting crystals were recrystallised from ethanol, to give the title compound, melting at 198-199°C, in a yield of 90%.

Infrared Absorption Spectrum (KBr) $\nu_{max}$ $cm^{-1}$:

3525, 3480, 3320, 1630.

Mass Spectrum m/e:

291 ($M^+$), 193.

EXAMPLE 23

2-(4-Acetyl-1-piperazinyl)-4-amino-6-ethylthieno[2,3-d]-pyrimidine, Compound No. 151

The procedure described in Example 22 was repeated, except that the Compound No. 3 was replaced by 4-amino-6-ethyl-2-(1-piperazinyl)thieno[2,3-d]pyrimidine (Compound No. 8), to give the title compound, melting at 180-181°C, in a yield of 79%.

Infrared Absorption Spectrum ($CHCl_3$) $\nu_{max}$ $cm^{-1}$:

3420, 1630, 1608, 1580.

Mass Spectrum m/e:

305 ($M^+$), 207.

EXAMPLE 24

4-Amino-6-ethyl-2-(tetrahydro-1,4-thiazin-4-yl)thieno[2,3-d]-pyrimidine, Compound No. 1

The procedure described in Example 1 was repeated, except that the 1-methylpiperazine was replaced by 0.85 g of thiomorpholine, to give 0.89 g (yield 80%) of the title compound, melting at 124-126°C.

Infrared Absorption Spectrum (KBr) $\nu_{max}$ $cm^{-1}$:

3470, 3300, 1630, 1580.

Mass Spectrum m/e:

280 ($M^+$), 207.

EXAMPLES 25 - 29

Following the procedure described in Example 1 or Example 3, but using the appropriate amines and/or thienopyrimidine derivatives, the following compounds were prepared.

EXAMPLE 25

4-Amino-5-ethyl-6-methyl-2-(4-methyl-1-piperazinyl)-thieno[2,3-d]pyrimidine hydrochloride (hydrochloride of Compound No. 226), melting at 292-294°C (with decomposition) in a yield of 85%.

EXAMPLE 26

4-Amino-2-[2-(diethylamino)ethylamino]-5-ethyl-6-methylthieno[2,3-d]pyrimidine (Compound No. 227), melting at 102-104°C, in a yield of 81%.

EXAMPLE 27

5-Ethyl-2-(4-formyl-1-piperazinyl)-6-methyl-4-methylaminothieno[2,3-d]pyrimidine (Compound No. 105), melting at 179-181°C, in a yield of 51%.

EXAMPLE 28

5-Ethyl-2-(4-formyl-1-piperazinyl)-4-(2-hydroxyethylamino)-6-methylthieno[2,3-d]pyrimidine (Compound No. 132), melting at 168-170°C, in a yield of 34%.

EXAMPLE 29

4-Amino-5-ethyl-2-(4-formyl-1-piperazinyl)-6-methylthieno[2,3-d]pyrimidine (Compound No. 89), melting at 228-230°C (with decomposition), in a yield of 43%.

EXAMPLES 30 - 32

The procedure described in Example 12 was repeated, except that the Compound No. 84 was replaced by Compound No. 105, 132 or 89, as prepared in Examples 27-29, respectively, and that, in Example 30, the product was first neutralized and then reacted with maleic acid, to give the following compounds:

EXAMPLE 30

5-Ethyl-6-methyl-4-methylamino-2-(1-piperazinyl)-thieno[2,3-d]pyrimidine maleate (maleate of Compound No. 27), melting at 180-182°C (with decomposition), in a yield of 96%.

EXAMPLE 31

5-Ethyl-4-(2-hydroxyethylamino)-6-methyl-2-(1-piperazinyl)thieno[2,3-d]pyrimidine (Compound No. 57), melting at 154-156°C, in a yield of 80%.

EXAMPLE 32

4-Amino-5-ethyl-6-methyl-2-(1-piperazinyl)thieno-[2,3-d]pyrimidine (Compound No. 13), melting at 300°C or above, in a yield of 83%.

EXAMPLE 33

4-Amino-2-(1-piperazinyl)thieno[2,3-d]pyrimidine, Compound No. 48

(a) To a saturated methanolic solution of ammonia were added 4.1 g of 4-amino-2,4-bis(methylthio)thieno[2,3-d]-pyrimidine (melting at 70-72°C). The mixture was then heated in a sealed tube at 200°C for 5 hours, after which the solvent was distilled off and the residue was recrystallized from methanol, to give 4-amino-2-methylthiothieno[2,3-d]pyrimidine, melting at 209-211°C, in a yield of 65%.

Infrared Absorption Spectrum (KBr) $\nu_{max}$ $cm^{-1}$:
3370, 3300, 3120, 1640.

(b) To 300 ml of chloroform was added 1 g of 4-amino-2-methylthiothieno[2,3-d]pyrimidine, and 1.9 g of m-chloroperbenzoic acid were added thereto. The mixture was stirred at room temperature overnight. The precipitate which was produced was collected by filtration and recrystallized from ethanol, to give the desired 4-amino-2-methylsulphonylthieno-[2,3-d]pyrimidine, melting at 248-250°C (with decomposition), in a yield of 65%.

Infrared Absorption Spectrum (KBr) $\nu_{max}$ $cm^{-1}$:
3440, 3310, 1650, 1638, 1302, 1130.

(c) To 2 ml of 1-benzylpiperazine was added 0.46 g of 4-amino-2-methylsulphonylthieno[2,3-d]pyrimidine and the mixture was heated in a sealed tube at 190°C for 7 hours, after which it was extracted with chloroform. The chloroform extract was washed with water and then subjected to chromatography through a column of silica gel eluted with 3% v/v ethanol in chloroform, to give 4-amino-2-(4-benzyl-1-piperazinyl)thieno[2,3-d]pyrimidine in a yield of 71%.

Infrared Absorption Spectrum ($CHCl_3$) $\nu_{max}$ $cm^{-1}$:
3530, 3420, 1610, 1580.

(d) To 20 ml of benzene was added 0.5g of 4-amino-2-([4-benzyl-1-piperazinyl)thieno[2,3-d]pyrimidine, and 0.8 ml of ethyl chlorocarbonate was added to the resulting mixture. The mixture was then refluxed for 6 hours, after which the resulting precipitate was collected by filtration and mixed with aqueous ammonia. The mixture was extracted with chloroform, giving 4-amino-2-(4-ethoxycarbonyl-1-piperazinyl)thieno[2,3-d]pyrimidine in a yield of 45%.

Infrared Absorption Spectrum ($CHCl_3$) $\nu_{max}$ $cm^{-1}$:

3540, 3430, 1665, 1610.

(e) To 2 ml of ethanol was added 0.25 g of 4-amino-2-(4-ethyoxycarbonyl-1-piperazinyl)thieno[2,3-d]pyrimidine, and 1 ml of a 50% w/v aqueous solution of potassium hydroxide was added thereto. The mixture was then heated under reflux for 6 hours, after which it was concentrated by evaporation under reduced pressure. The residue was diluted with water and extracted with chloroform, to give the title compound in a yield of 58%. This was then reacted with hydrochloric acid, to give the hydrochloride, melting at 265-270°C (with decomposition).

Infrared Absorption Spectrum of hydrochloride (KBr) $\nu_{max}$ $cm^{-1}$:

3500, 3340, 1615.

EXAMPLES 34-37

Following the procedure described in Example 1 or Example 3 and using the products of Preparations 14-17 and the appropriate amines, the following compounds were prepared:

EXAMPLE 34

4-Amino-2-(4-formyl-1-piperazinyl)-6-methylthieno-[2,3-d]pyrimidine (Compound No. 83), melting at 212-213°C, in a yield of 35%.

EXAMPLE 35

2-(4-Formyl-1-piperazinyl)-6-methyl-4-methylaminothieno-[2,3-d]pyrimidine (Compound No. 98), melting at 167-169°C, in a yield of 57%.

EXAMPLE 36

6-Ethyl-4-ethylamino-2-(4-formyl-1-piperazinyl)thieno-[2,3-d]pyrimidine (Compound No. 114), melting at 185-187°C, in a yield of 53%.

EXAMPLE 37

4-Amino-2-(4-formyl-1-piperazinyl)-6-isopropylthieno-[2,3-d]pyrimidine (Compound No. 228), melting at 212-214°C, in a yield of 52%.

EXAMPLES 38-41

The procedure described in Example 12 was repeated, but using the products of Examples 34-37, respectively, to give the following compounds:

EXAMPLE 38

4-Amino-6-methyl-2-(1-piperazinyl)thieno[2,3-d]-pyrimidine hydrochloride (hydrochloride of Compound No. 7), melting at 294-296°C (with decomposition), in a yield of 68%.

EXAMPLE 39

6-Methyl-4-methylamino-2-(1-piperazinyl)thieno[2,3-d]-pyrimidine hydrochloride (hydrochloride of Compound No. 20), melting at 303-305°C (with decomposition), in a yield of 81%.

EXAMPLE 40

6-Ethyl-4-ethylamino-2-(1-piperazinyl)thieno)[2,3-d]-pyrimidine hydrochloride (hydrochloride of Compound No. 36

melting at 269-271°C (with decomposition), in a yield of 60%.

EXAMPLE 41

4-Amino-6-isopropyl-2-(1-piperazinyl)thieno[2,3-d]-pyrimidine (Compound No. 229), melting at 197-199°C, in a yield of 95%.

EXAMPLES 42-47

(a) Following the procedure described in Example 1 or Example 3, but using the appropriate amines and thienopyrimidine derivatives, the following compounds were prepared:

EXAMPLE 42

2-(4-Ethoxycarbonyl-1-piperazinyl)-6-isopropyl-4-methylaminothieno[2,3-d]pyrimidine, melting at 147-148°C in a yield of 82%.

EXAMPLE 43

2-(4-Isobutoxycarbonyl-1-piperazinyl)-5-methyl-4-methylaminothieno[2,3-d]pyrimidine, melting at 148-149°C, in a yield of 89%.

EXAMPLE 44

4-Amino-2-(4-ethoxycarbonyl-1-piperazinyl)-5-isopropylthieno[2,3-d]pyrimidine, melting at 132-134°C, in a yield of 74%.

EXAMPLE 45

2-(4-Ethoxycarbonyl-1-piperazinyl)-5-isopropyl-4-methylaminothieno[2,3-d]pyrimidine, melting at 175-177°C, in a yield of 90%.

EXAMPLE 46

4-Butylamino-2-(4-ethoxycarbonyl-1-piperazinyl)-5-methylthieno[2,3-d]pyrimidine, melting at 97-99°C, in a yield of 67%.

EXAMPLE 47

2-(4-Ethoxycarbonyl-1-piperazinyl)-4-ethylamino-5-methylthieno[2,3-d]pyrimidine, melting at 128-130°C, in a yield of 75%.

(b) The procedure described in Example 33 (e) was repeated, but using the compounds obtained as described

in Examples 42-47 (a), to give the following compounds:

EXAMPLE 42

6-Isopropyl-4-methylamino-2-(1-piperazinyl)thieno-[2,3-d]pyrimidine hydrochloride (hydrochloride of Compound No. 230), melting at 241-243°C (with decomposition), in a yield of 98%.

EXAMPLE 43

5-Methyl-4-methylamino-2-(1-piperazinyl)thieno-[2,3-d]pyrimidine hydrochloride (hydrochloride of Compound No. 16), melting at 296-299°C (with decomposition), in a yield of 75%.

EXAMPLE 44

4-Amino-5-isopropyl-2-(1-piperazinyl)thieno[2,3-d]-pyrimidine hydrochloride (hydrochloride of Compound No. 6), melting at 271-274°C (with decomposition), in a yield of 70%.

EXAMPLE 45

5-Isopropyl-4-methylamino-2-(1-piperazinyl)thieno-[2,3-d]pyrimidine hydrochloride (hydrochloride of Compound No. 19).

melting at 251-253°C (with decomposition), in a yield of 74%.

EXAMPLE 46

4-Butylamino-5-methyl-2-(1-piperazinyl)thieno[2,3-d]-pyrimidine hydrochloride (hydrochloride of Compound No. 231), melting at 223-225°C (with decomposition), in a yield of 83%.

EXAMPLE 47

4-Ethylamino-5-methyl-2-(1-piperazinyl)thieno[2,3-d]-pyrimidine hydrochloride (hydrochloride of Compound No. 33), melting at 247-249°C (with decomposition), in a yield of 98%.

EXAMPLE 48

4-Methylamino-2-(1-piperazinyl)thieno[2,3-d]pyrimidine, Compound No. 48

(a) The procedure described in Example 1 was repeated, except that 2-chloro-4-methylaminothieno[2,3-d]-pyrimidine was used in place of the 4-amino-2-chloro-6-ethylthieno[2,3-d]pyrimidine and that 1-isobutoxycarbonyl-piperazine was used in place of the 1-methylpiperazine,

to give 2-(4-isobutoxycarbonyl-1-piperazinyl)-4-methylaminothieno-[2,3-d]pyrimidine, melting at 140-145°C, in a yield of 90%.

(b) The procedure described in Example 33 (e) was repeated, but using the compound obtained in step (a) above, to give the title compound and then this was converted to the hydrochloride by reaction with hydrochloric acid, to give the hydrochloride, melting at 281-284°C (with decomposition), in an overall yield of 90%.

Infrared Absorption Spectrum of hydrochloride (KBr) $\nu_{max}$ $cm^{-1}$: 3410, 1625, 1595.

## EXAMPLE 49

4-Amino-2-(4-methyl-1-piperazinyl)-5,6-tetramethylenethieno-[2,3-d]pyrimidine, Compound No. 203

1.5 g of 4-amino-2-chloro-5,6-tetramethylenethieno-[2,3-d]pyrimidine and 2 ml of 1-methylpiperazine were heated under reflux with 10 ml of isoamyl alcohol for 7 hours. The resulting precipitate was collected by filtration and recrystallised from ethanol to give 1.52 g (yield 80%) of the title compound, which was then converted to

the hydrochloride by reaction with hydrochloric acid.

The hydrochloride melted at 305-306°C (with decomposition) and its infrared absorption spectrum (KBr) was, $\nu_{max}$ $cm^{-1}$:

3450, 3300, 3210, 1620.

## EXAMPLES 50 - 54

The procedure described in Example 49 was repeated, except that different thienopyrimidine derivatives were used and/or the 1-methylpiperazine was replaced by thiomorpholine, to give the following compounds:

### EXAMPLE 50

4-(2-Hydroxyethylamino)-2-(4-methyl-1-piperazinyl)-5,6-tetramethylenethieno[2,3-d]pyrimidine hydrochloride (hydrochloride of Compound No. 205), melting at 207-209°C (with decomposition), in a yield of 62%.

Infrared Absorption Spectrum (KBr) $\nu_{max}$ $cm^{-1}$:

3400, 1610, 1590.

EXAMPLE 51

4-Methylamino-2-(4-methyl-1-piperazinyl)-5,6-tetramethylenethieno[2,3-d]pyrimidine hydrochloride (hydrochloride of Compound No. 232), melting at 291-293°C (with decomposition), in a yield of 82%.

Infrared Absorption Spectrum (KBr) $\nu_{max}$ $cm^{-1}$:
3300, 1625, 1588.

EXAMPLE 52

4-Amino-2-(tetrahydro-1,4-thiazin-4-yl)-5,6-trimethylenethieno[2,3-d]pyrimidine hydrochloride (hydrochloride of Compound No. 178), melting at 241-243°C (with decomposition), in a yield of 84%.

Infrared Absorption Spectrum (KBr) $\nu_{max}$ $cm^{-1}$:
3300, 3110, 1650, 1600.

EXAMPLE 53

4-Amino-2-(tetrahydro-1,4-thiazin-4-yl)-5,6-tetramethylenethieno[2,3-d]pyrimidine hydrochloride (hydrochloride of Compound No. 180), melting at 212-214°C, in

a yield of 84%.

Infrared Absorption Spectrum (KBr) $\nu_{max}$ $cm^{-1}$:

3400, 3280, 1635, 1590.

EXAMPLE 54

4-Amino-2-(tetrahydro-1,4-thiazin-4-yl)-5,6-pentamethylenethieno[2,3-d]pyrimidine hydrochloride (hydrochloride of Compound No. 182), melting at 234-236°C, in a yield of 95%.

Infrared Absorption Spectrum (KBr) $\nu_{max}$ $cm^{-1}$:

3320, 3080, 1638, 1600.

EXAMPLES 55 - 59

The procedure described in Example 49 was repeated, but replacing the 1-methylpiperazine by 1-formylpiperazine and, in some cases, using different thienopyrimidine derivatives; the conversion to the hydrochloride was also omitted, to give the following compounds:

EXAMPLE 55

4-Amino-2-(4-formyl-1-piperazinyl)-5,6-tetramethylene-thieno[2,3-d]pyrimidine (Compound No. 208), melting at 234-235°C, in a yield of 35%.

Infrared Absorption Spectrum (KBr) $\nu_{max}$ $cm^{-1}$:
3530, 3340, 3220, 1670, 1628.

EXAMPLE 56

4-Amino-2-(4-formyl-1-piperazinyl)-5,6-trimethylene-thieno[2,3-d]pyrimidine (Compound No. 207), melting at 229-231°C, in a yield of 52%.

Infrared Absorption Spectrum (KBr) $\nu_{max}$ $cm^{-1}$:
3450, 3300, 3220, 1658, 1620.

EXAMPLE 57

4-Amino-2-(4-formyl-1-piperazinyl)-5,6-pentamethylene-thieno[2,3-d]pyrimidine (Compound No. 209), melting at 243-244°C, in a yield of 44%.

Infrared Absorption Spectrum (KBr) $\nu_{max}$ $cm^{-1}$:
3520, 3310, 3220, 1665, 1630.

EXAMPLE 58

2-(4-Formyl-1-piperazinyl)-4-(2-hydroxyethylamino-5,6-tetramethylenethieno[2,3-d]pyrimidine (Compound No. 215), melting at 189-191°C, in a yield of 29%.

Infrared Absorption Spectrum (KBr) $\nu_{max}$ $cm^{-1}$:
3430, 1660, 1575.

EXAMPLE 59

4-Bis(2-hydroxyethyl)amino-2-(4-formyl-1-piperazinyl)-5,6-tetramethylenethieno[2,3-d]pyrimidine (Compound No. 218), melting at 146-147°C, in a yield of 24%.

Infrared Absorption Spectrum (KBr) $\nu_{max}$ $cm^{-1}$:
3320, 1680, 1585.

EXAMPLES 60 - 62

4-Amino-2-(1-piperazinyl)-5,6-tetramethylenethieno[2,3-d]-pyrimidine, Compound No. 185

In these Examples, the title compound was prepared, in the form of its dihydrochloride, by three different methods.

EXAMPLE 60

Deformylation

To 8 ml of 10% w/v hydrochloric acid was added 0.8 g of 4-amino-2-(4-formyl-1-piperazinyl)-5,6-tetramethylenethieno[2,3-d]pyrimidine (Compound No. 208, prepared as described in Example 55). The mixture was heated under reflux for 1 hour, after which it was concentrated by evaporation under reduced pressure and ethanol was added to the concentrate. The resulting precipitate was collected by filtration, giving the dihydrochloride of the title compound, melting at 300°C or above, in a yield of 79%.

Infrared Absorption Spectrum (KBr) $\nu_{max}$ $cm^{-1}$:

3470, 3330, 3220, 1615.

EXAMPLE 61

Dealkoxycarbonylation

(a) The procedure described in Example 49 was

repeated, except that the 1-methylpiperazine was replaced by ethyl piperazine-1-carboxylate and the conversion to the hydrochloride was omitted, to give 4-amino-2-(4-ethoxycarbonyl-1-piperazinyl)-5,6-tetramethylenethieno[2,3-d]-pyrimidine, melting at 243-244°C, in a yield of 45%.

(b) To 2 ml of ethanol was added 1.0 g of 4-amino-2-(4-ethoxycarbonyl-1-piperazinyl)-5,6-tetramethylenethieno-[2,3-d]pyrimidine, prepared as described in step (a) above, followed by 1 ml of a 50% w/v aqueous solution of potassium hydroxide. The mixture was heated under reflux for 5 hours, after which it was concentrated by evaporation under reduced pressure and the concentrate was diluted with water. The resulting precipitate was collected by filtration and converted to the dihydrochloride by reaction with hydrochloric acid. The yield of dihydrochloride was 58%.

EXAMPLE 62

Direct reaction with piperazine

1.5 g of 4-amino-2-chloro-5,6-tetramethylenethieno-[2,3-d]pyrimidine and 3.14 g of piperazine hydrobromide were refluxed with 20 ml of 2-methoxyethanol for 8 hours.

The product was collected by filtration and suspended in water. The pH of the suspension was adjusted to a value of 12, and then the suspension was extracted with chloroform. The extract was subjected to chromatography through silica gel eluted with a 1:4 by volume mixture of ethanol and chloroform, to give the title compound, which was converted to the dihydrochloride by reaction with hydrochloric acid. The yield was 34%.

## EXAMPLE 63

4-Amino-2-(2-aminoethylamino)-5,6-tetramethylenethieno[2,3-d]-pyrimidine, Compound, No. 200

(a) The procedure described in Example 49 was repeated, except that the 1-methylpiperazine was replaced by 2-acetylaminoethylamine and that the conversion to hydrochloride was omitted, to give 2-(2-acetylaminoethylamino)-4-amino-5,6-tetramethylenethieno[2,3-d]pyrimidine, melting at 229-231°C (with decomposition), in a yield of 56%.

(b) Employing the compound prepared as described in step (a) above, the procedure described in Example 60 was repeated, to give the title compound, in the form

of its hydrochloride melting at 306-307°C (with decomposition), in a yield of 60%.

Infrared Absorption Spectrum (KBr) $\nu_{max}$ $cm^{-1}$:

3470, 3340, 3200, 1615.

EXAMPLES 64 - 67

The procedure described in Example 60 was repeated, except that the starting materials were the products of Examples 58, 56, 57 and 59, respectively, to give the following products:

EXAMPLE 64

4-(2-Hydroxyethylamino)-2-(1-piperazinyl)-5,6-tetramethylenethieno[2,3-d]pyrimidine hydrochloride (hydrochloride of Compound No. 194), melting at 215-217°C (with decomposition), in a yield of 40%.

Infrared Absorption Spectrum (KBr) $\nu_{max}$ $cm^{-1}$:

3370, 3320, 1610, 1590.

EXAMPLE 65

4-Amino-2-(1-piperazinyl)-5,6-trimethylenethieno-[2,3-d]pyrimidine hydrochloride (hydrochloride of Compound No. 184), melting at 212-213°C (with decomposition), in a yield of 40%.

Infrared Absorption Spectrum (KBr) $\nu_{max}$ $cm^{-1}$:

3580, 3340, 1610, 1570.

EXAMPLE 66

4-Amino-2-(1-piperazinyl)-5,6-pentamethylenethieno-[2,3-d]pyrimidine hydrochloride (hydrochloride of Compound No. 186), melting at 300°C or above, in a yield of 74%.

Infrared Absorption Spectrum (KBr) $\nu_{max}$ $cm^{-1}$:

3430, 1637, 1605.

EXAMPLE 67

4-Bis(2-hydroxyethyl)amino-2-(1-piperazinyl)-5,6-tetramethylenethieno[2,3-d]pyrimidine (Compound No. 197), melting at 121-123°C, in a yield of 63%.

Infrared Absorption Spectrum (KBr) $\nu_{max}$ $cm^{-1}$:

3430, 3240, 1580.

## EXAMPLE 68

4-Methylamino-2-(1-piperazinyl)-5,6-tetramethylenethieno-[2,3-d]pyrimidine, Compound No. 188

The procedure described in Example 62 was repeated, except that there were used 1.52 g of 2-chloro-4-methylamino-5,6-tetramethylenethieno[2,3-d]pyrimidine and 1.0 g of piperazine hydrobromide and that conversion to the hydrochloride was omitted, to give the title compound, melting at 181-183°C, in a yield of 44%.

Infrared Absorption Spectrum (KBr) $\nu_{max}$ $cm^{-1}$:

3480, 3430, 3310, 3270, 1580.

CLAIMS:

1. Compounds of formula (I):

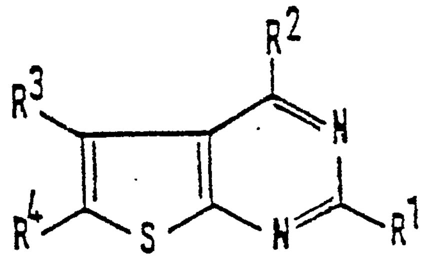

(I)

wherein:

$R^1$ represents an aminoalkylamino group of formula

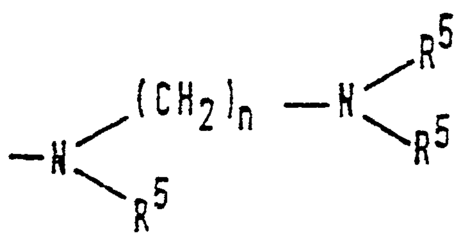

or a bis(aminoalkyl)amino group of formula

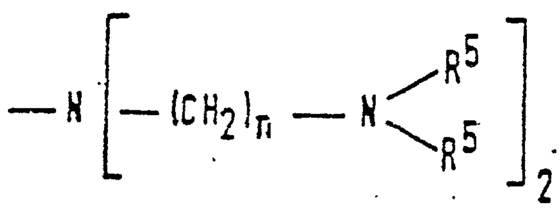

or a heterocyclic group of formula

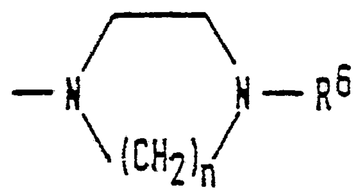

(in which: the symbols $R^5$ may be the same or different and each represents a hydrogen atom or a $C_1$-$C_4$ alkyl group, $R^6$ represents a hydrogen atom, a $C_1$-$C_4$ alkyl group, a formyl group or a $C_2$-$C_4$ alkanoyl group and _n_ is 2 or 3)

or a tetrahydro-1,4-thiazin-4-yl group;

$R^2$ represents an amino group of formula 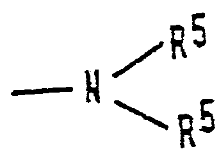

(in which the symbols $R^5$, which may be the same or different, are as defined above), a 2-hydroxyethylamino group, a bis(2-hydroxyethyl)amino group or an aminoethylamino group of formula

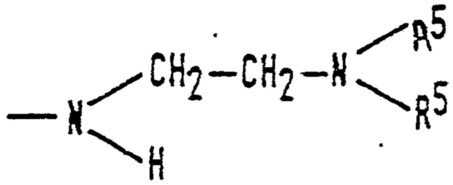

 

(in which the symbols $R^5$, which may be the same or different, are as defined above); and

$R^3$ and $R^4$ are the same or different and each represents a hydrogen atom or a $C_1$-$C_4$ alkyl group or $R^3$ and $R^4$ together represent a polymethylene group of formula $-(CH_2)_p-$ (in which p is an integer from 3 to 5);

and pharmaceutically acceptable acid addition salts thereof.

2. Compounds as claimed in Claim 1, wherein $R^1$ represents said aminoalkylamino group or said bis(aminoalkyl)-amino group in which the symbols $R^5$ are the same or different and each represents a hydrogen atom, a methyl group or an ethyl group and n is 2.

3. Compounds as claimed in Claim 1, wherein $R^1$ represents said heterocyclic group in which n is 2 and $R^6$ represents a hydrogen atom, a methyl group, an ethyl group, a formyl group or an acetyl group.

4. Compounds as claimed in Claim 1, wherein $R^1$ represents said heterocyclic group in which n is 2 and $R^6$ represents a hydrogen atom, a methyl group or a formyl group.

5. Compounds as claimed in Claim 1, wherein $R^1$ represents said tetrahydro-1,4-thiazin-4-yl group.

6. Compounds as claimed in any one of the preceding Claims, wherein $R^2$ represents a group of formula $-NHR^5$ (in which $R^5$ represents a hydrogen atom or a $C_1$-$C_4$ alkyl group), a 2-hydroxyethylamino group, a bis(2-hydroxyethyl)amino group or a 2-aminoethylamino group.

7. Compounds as claimed in Claim 6, wherein $R^2$ represents an amino group, a methylamino group, an ethylamino group, a 2-hydroxyethylamino group, a bis(2-hydroxyethyl)-amino group or a 2-aminoethylamino group.

8. Compounds as claimed in any one of the preceding Claims, wherein one of $R^3$ and $R^4$ represents a hydrogen atom and the other represents a hydrogen atom or a $C_1$-$C_3$ alkyl group.

9. Compounds as claimed in Claim 8, wherein $R^3$ represents a hydrogen atom and $R^4$ represents a methyl group or an ethyl group.

10. Compounds as claimed in Claim 8, wherein $R^3$ represents a methyl group and $R^4$ represents a hydrogen atom.

11. Compounds as claimed in Claim 8, wherein $R^3$ and $R^4$ both represent hydrogen atoms.

12. Compounds as claimed in any one of Claims 1 to 7, wherein $R^3$ and $R^4$ together represent a tetramethylene or pentamethylene group.

13. 4-Amino-5-methyl-2-(1-piperazinyl)thieno[2,3-d]-pyrimidine and pharmaceutically acceptable acid addition salts thereof.

14. 4-Amino-6-ethyl-2-(1-piperazinyl)thieno[2,3-d]-pyrimidine and pharmaceutically acceptable acid addition salts thereof.

15. 5-Methyl-4-methylamino-2-(1-piperazinyl)thieno[2,3-d]-pyrimidine and pharmaceutically acceptable acid addition salts thereof.

16. 4-Amino-2-(1-piperazinyl)thieno[2,3-d]pyrimidine and pharmaceutically acceptable acid addition salts thereof.

17. 4-Methylamino-2-(1-piperazinyl)thieno[2,3-d]pyrimidine and pharmaceutically acceptable acid addition salts thereof.

18. 6-Ethyl-4-(2-hydroxyethylamino)-2-(1-piperazinyl)-thieno[2,3-d]pyrimidine and pharmaceutically acceptable acid addition salts thereof.

19. 4-Amino-2-(tetrahydro-1,4-thiazin-4-yl)-5,6-pentamethylenethieno[2,3-d]pyrimidine and pharmaceutically acceptable acid addition salts thereof.

20. 4-Methylamino-2-(1-piperazinyl)-5,6-trimethylene-thieno[2,3-d]pyrimidine and pharmaceutically acceptable acid addition salts thereof.

21. 4-Bis(2-hydroxyethyl)amino-2-(1-piperazinyl)-5,6-tetramethylenethieno[2,3-d]pyrimidine and pharmaceutically acceptable acid addition salts thereof.

22. The use of a compound as claimed in any one of Claims 1 to 21 for the treatment of thrombosis, hyperglycaemia, hypertension or oedema.

23. A process for preparing a compound as claimed in any one of Claims 1 to 21, which process comprises:

(a) reacting a compound of formula (II):

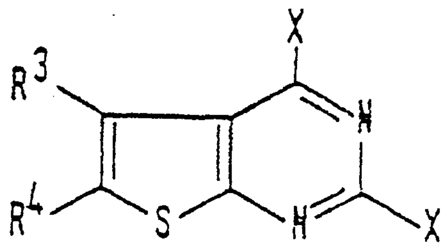

(II)

(in which $R^3$ and $R^4$ are as defined in Claim 1 and X represents a halogen atom, a lower alkylsulphinyl group, an arylsulphinyl group, a lower alkylsulphonyl group or an arylsulphonyl group) with a compound of formula $R^2H$ (in which $R^2$ is as defined in Claim 1), to give a compound of formula (III):

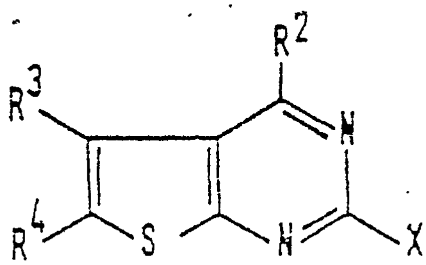

(III)

(in which $R^2$, $R^3$ and $R^4$ are as defined in Claim 1 and X is as defined above);

(b) reacting said compound of formula (III) with a compound of formula $R^7H$ (in which $R^7$ represents any one of the groups defined for $R^1$, in which any free

amino group is optionally protected), to give a compound of formula (IV):

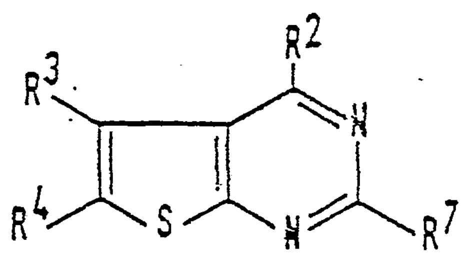

(IV)

(in which $R^2$, $R^3$ and $R^4$ are as defined in Claim 1 and $R^7$ is as defined above), provided that, where $R^2$ and $R^7$ represent the same groups, the reactions of steps (a) and (b) may be carried out as a single step or as two separate steps;

(c) if necessary, removing any amino-protecting groups to give a compound of formula (I) and, where $R^6$ in said compound of formula (I) represents a hydrogen atom, optionally acylating said compound to give a compound of formula (I) in which $R^6$ represents said formyl or $C_2$-$C_4$ alkanoyl group; and

(d) if necessary, salifying the resulting compound of formula (I).

24. A process as claimed in Claim 23, wherein step (a) is effected in an inert solvent and in the presence of a base.

25. A process as claimed in Claim 23 or Claim 24, in which step (a) is effected at a temperature of from -10°C to +40°C.

26. A process as claimed in any one of Claims 23 to 25, in which step (b) is effected in an inert solvent and in the presence of a base.

27. A process as claimed in any one of Claims 23 to 26, in which step (b) is effected at a temperature of from 0°C to 300°C.

28. A process according to Claim 23, in which the reactions of steps (a) and (b) are effected in a single stage, in an inert solvent and in the presence of a base, and at a temperature of from 0°C to 300°C.